(19) Europäisches Patentamt
European Patent Office
Office européen des brevets

(11) **EP 4 015 525 A2**

(12) **EUROPEAN PATENT APPLICATION**

(43) Date of publication:
**22.06.2022 Bulletin 2022/25**

(21) Application number: **21194817.9**

(22) Date of filing: **25.03.2019**

(51) International Patent Classification (IPC):
**C07K 14/435** (2006.01)    **C07K 16/00** (2006.01)
**C12N 5/0783** (2010.01)    **A61P 35/00** (2006.01)
**C07K 14/705** (2006.01)    **C07K 14/725** (2006.01)
**C07K 16/28** (2006.01)    **C07K 16/30** (2006.01)

(52) Cooperative Patent Classification (CPC):
**C12N 5/0636; A61P 35/00; C07K 14/705;**
**C07K 14/7051; C07K 14/70517; C07K 16/2803;**
**C07K 16/3084;** C07K 2317/622; C07K 2319/03;
C07K 2319/33; C12N 2510/00

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **23.03.2018 GB 201804701**

(62) Document number(s) of the earlier application(s) in accordance with Art. 76 EPC:
**19713774.8 / 3 768 696**

(71) Applicant: **GammaDelta Therapeutics Limited**
**London W12 7FQ (GB)**

(72) Inventors:
• **NUSSBAUMER, Oliver**
**London, NW1 0NH (GB)**
• **KOVÁCS, István**
**London, NW1 0NH (GB)**

• **PIZZITOLA, Irene**
**London, NW1 0NH (GB)**
• **MEHTA, Raj**
**London, NW1 0NH (GB)**

(74) Representative: **Mewburn Ellis LLP**
**Aurora Building**
**Counterslip**
**Bristol BS1 6BX (GB)**

Remarks:
•This application was filed on 03-09-2021 as a divisional application to the application mentioned under INID code 62.
•Claims filed after the date of filing of the application / after the date of receipt of the divisional application (Rule 68(4) EPC).

(54) **LYMPHOCYTES EXPRESSING HETEROLOGOUS TARGETING CONSTRUCTS**

(57) The present invention provides engineered lymphocytes (e.g., $\gamma\delta$ T cells, NK cells, NK-like T cells, engineered innate lymphoid cells, or MAIT cells) comprising a heterologous targeting construct lacking an intracellular signaling domain capable of activating the lymphocyte on which the construct is expressed. Further provided are compositions of engineered lymphocytes (e.g., $\gamma\delta$ T cells) and methods of using the engineered lymphocytes (e.g., $\gamma\delta$ T cells, e.g., a part of an adoptive T cell therapy).

EP 4 015 525 A2

**Description**

**BACKGROUND**

Cancer is a group of diseases involving abnormal cell growth with the potential to metastasize to other parts of the body. The diversity of types of cancers is well-known, and many types of cancers can drastically vary in their genetic makeup between patients. This variation creates a difficult burden in identifying effective therapeutic strategies for targeting certain cancers. In particular, a need exists to create personalized therapeutic strategies to any given cancer target. As a result, a growing interest in T cell immunotherapy has emerged based on the identification that we can harness cells of the immune system to recognize and destroy foreign or pathogenic cells. To date, T cell immunotherapies have involved engineering $\alpha\beta$ T cells to express chimeric antigen receptors (CARs). Such CAR T cells can identify a cancer target based on expression of a target antigen (e.g., a tumor-associated antigen) recognized by the chimeric antigen receptor. Upon binding to its target antigen, one or more intracellular domains of the CAR propagate signal 1 activation and/or signal 2 activation (co-stimulation) to activate the CAR T cell, thereby triggering degranulation and lysis of the target cell. However, several problems remain with such CAR T cell approaches. For example, CAR T cells run the risk of conferring off-target cytotoxicity due to moderate expression of target antigen by healthy cells. Accordingly, there is a need in the field for improved methods to engineer these powerful components of the immune system while enhancing safety and efficacy of the treatment.

**SUMMARY OF THE INVENTION**

The present invention provides an alternative approach to CAR T cells. Specifically, featured herein are heterologous targeting constructs that lack a functional intracellular domain capable of activating the cell on which it is expressed. When expressed on lymphocytes having innate-like effector functions and/or are not MHC-restricted, such as $\gamma\delta$ T cells, NK cells, NK-like T cells, innate lymphoid cells, and engineered mucosal-associated invariant T (MAIT) cells, the engineered lymphocyte can exhibit enhanced specificity to diseased cells by avoiding aberrant TCR activation upon binding to low levels of target antigen on healthy cells.

In a first aspect, the invention features an engineered gamma-delta ($\gamma\delta$) T cell including a heterologous targeting construct, wherein the heterologous targeting construct includes an extracellular antigen-binding domain and a transmembrane domain operatively linked to the antigen-binding domain, wherein the heterologous targeting construct lacks an intracellular domain capable of activating the engineered $\gamma\delta$ T cell (e.g., the intracellular domain, if present, does not propagate signal 1 activation and does not propagate signal 2 co-stimulation). In some embodiments, the heterologous targeting construct further includes a stalk domain operatively linking the antigen-binding domain to the transmembrane domain.

In another aspect, the invention provides an engineered $\gamma\delta$ T cell including a heterologous targeting construct, wherein the heterologous targeting construct includes an antigen-binding domain and a transmembrane domain, wherein the transmembrane domain is a terminal transmembrane domain (i.e., a transmembrane domain having an unlinked terminal end, e.g., a C-terminus that is not linked to a peptide or protein). Thus, a terminal transmembrane domain is not linked to an intracellular domain, such as an intracellular signaling domain. The transmembrane domain does not propagate signal 1 activation. In some embodiments, a terminal transmembrane domain does not participate in an intracellular signaling pathway (e.g., a TCR pathway, e.g., a T cell signaling pathway, such as signal 2 co-stimulation). In other embodiments, the transmembrane domain may associate with endogenous molecules, thereby propagating signal 2 co-stimulation. In some embodiments, the heterologous targeting construct further includes a stalk domain operatively linking the antigen-binding domain to the transmembrane domain.

In some embodiments of any aspect of the invention, the transmembrane domain does not activate the engineered $\gamma\delta$ T cell.

In another aspect, the invention features an engineered $\gamma\delta$ T cell including a heterologous targeting construct consisting of an antigen-binding domain, a stalk domain operatively linked the antigen-binding domain, and a transmembrane domain operatively linked to the stalk domain.

In some embodiments of any aspect of the invention, the engineered $\gamma\delta$ T cell is V$\delta$2-negative (e.g., the V$\delta$2-negative $\gamma\delta$ T cell is V$\delta$1-positive or double negative). In alternative embodiments of any aspect of the invention, the engineered $\gamma\delta$ T cell can be V$\delta$2-positive.

The antigen-binding domain may include a single chain variable fragment (scFv), a monoclonal antibody, a Fab fragment, a B cell receptor, a T cell receptor, an antibody scaffold, a receptor-specific ligand, or a ligand-specific receptor (e.g., a receptor specific to a surface-expressed ligand). In some embodiments, the stalk domain includes one or more of the domains selected from the group consisting of a CD8 stalk, an IgG1 hinge-$CH_2$ domain, an IgG1-hinge-$CH_3$ domain, an IgG1-hinge-$CH_2$-$CH_3$ domain, a $(G_4S)_3$ hinge, an IgG1 hinge, a CD7 stalk, an IgD hinge, an IgD hinge-$CH_2$ domain, an IgD hinge-$CH_3$ domain, an IgD hinge-$CH_2$-$CH_3$ domain, an IgG4 hinge, an IgG4 hinge-$CH_2$ domain,

an IgG4 hinge-CH$_3$ domain, an IgG4 hinge-CH$_2$-CH$_3$ domain, or an FcεRI stalk domain.

In some embodiments of any aspect of the invention, the transmembrane domain includes a CD8 transmembrane domain, a CD4 transmembrane domain, a CD3ε transmembrane domain, a CD3ζ transmembrane domain, a CD28 transmembrane domain, a CD45 transmembrane domain, a CD5 transmembrane domain, a CD8 transmembrane domain, a CD9 transmembrane domain, a CD16 transmembrane domain, a CD22 transmembrane domain, a CD33 transmembrane domain, a CD37 transmembrane domain, a CD64 transmembrane domain, a CD80 transmembrane domain, a CD86 transmembrane domain, a CD134 transmembrane domain, a CD137 transmembrane domain, a CD154 transmembrane domain, a CD7 transmembrane domain, a CD71 transmembrane domain, a CD18 transmembrane domain, a CD29 transmembrane domain, a CD11a transmembrane domain, a CD11b transmembrane domain, a CD11c transmembrane domain, a CD11d transmembrane domain, a CD94 transmembrane domain, an FcγR transmembrane domain, or an NKG2D transmembrane domain. In some embodiments, no more than 50% of the amino acids of the terminal transmembrane domain reside intracellularly (e.g., no more than 45%, 40%, 35%, 30%, 25%, 20%, 15%, 10%, or 5% of the amino acids of the terminal transmembrane domain (e.g., C-terminal transmembrane domain) reside intracellularly).

In some embodiments of any aspect of the invention, clustering of the heterologous targeting construct upon binding of the antigen-binding domain to a target antigen does not substantially activate the TCR pathway in the engineered γδ T cell.

In some embodiments of any aspect of the invention, the antigen-binding domain binds a tumor-associated antigen. For example, the tumor-associated antigen may be a protein or peptide antigen expressed on the surface of a tumor cell (e.g., CD19). Alternatively, the tumor-associated antigen can be a carbohydrate expressed on the surface of a tumor cell. In some embodiments, the tumor-associated antigen is ganglioside expressed on the surface of a tumor cell (e.g., GD2). In some embodiments, the tumor-associated antigen is an immunosuppressive antigen. In one embodiment, the antigen-binding domain binds a target antigen that is expressed by a solid tumor cell.

In some of any of the preceding embodiments, the binding of the antigen-binding domain to a target antigen expressed on a healthy cell triggers substantially less cytolysis (e.g., at least 5% less, at least 10% less, at least 20% less, at least 30% less, at least 40% less, at least 50% less, at least 60% less, at least 70% less, at least 80% less, at least 90% less, or at least 95% less cytolysis) by the engineered γδ T cell relative to a reference cell having a functional intracellular domain (e.g., it does not substantially trigger cytolysis by the engineered γδ T cell). In some embodiments, binding of the antigen-binding domain to a target antigen expressed on a tumor cell or an infected cell substantially triggers cytolysis by the engineered γδ T cell. The cytolysis can be dependent on endogenous expression of NKG2D, NKp30, NKp44, NKp46, or DNAM1 by the engineered γδ T cell. In some embodiments, the cytolysis is characterized by one, two, three, four, five, or all six of the responses selected from the group consisting of CD107 degranulation, granzyme release, perforin release, granulysin release, target cell killing, proliferation of the γδ T cell, and cytokine production.

In another aspect, the invention features an engineered NK cell or NK-like T cell having a heterologous targeting construct of any of the embodiments described herein. In some embodiments, the heterologous targeting construct includes an extracellular antigen-binding domain and a transmembrane domain operatively linked to the antigen-binding domain. The heterologous targeting construct lacks an intracellular domain capable of activating the engineered NK cell or NK-like T cell.

In another aspect, the invention features an engineered innate lymphoid cell (ILC). The engineered ILC includes a heterologous targeting construct of any of the embodiments described herein. In some embodiments, the heterologous targeting construct includes an extracellular antigen-binding domain and a transmembrane domain operatively linked to the antigen-binding domain. The heterologous targeting construct lacks an intracellular domain capable of activating the engineered innate lymphoid cell.

In another aspect, the invention features an engineered MAIT cell. The engineered MAIT cell includes a heterologous targeting construct of any of the embodiments described herein. In some embodiments, the heterologous targeting construct includes an extracellular antigen-binding domain and a transmembrane domain operatively linked to the antigen-binding domain. The heterologous targeting construct lacks an intracellular domain capable of activating the engineered MAIT cell.

In another aspect, the invention features an isolated cell population that includes at least ten engineered γδ T cells, engineered NK cells or NK-like T cells, engineered innate lymphoid cells, or engineered MAIT cells of any of the preceding embodiments. In some embodiments, the engineered γδ T cells, engineered NK cells or NK-like T cells, engineered innate lymphoid cells, or engineered MAIT cells represent greater than 2% (e.g., between 2% and 100%, between 10% and 95%, between 20% and 90%, between 30% and 80%, between 40% and 70%, e.g., greater than 5%, greater than 10%, greater than 15%, greater than 20%, greater than 30%, greater than 40%, greater than 50%, greater than 60%, greater than 70%, greater than 80%, greater than 90%, greater than 95%, 96%, 97%, 98%, or 99%) of the total number of cells in the isolated cell population.

In another aspect, the invention features an isolated cell population that includes a plurality of engineered γδ T cells, NK cells, NK-like T cells, innate lymphoid cells, or MAIT cells of any one of the preceding embodiments. The population of the engineered γδ T cells, NK cells, NK-like T cells, innate lymphoid cells, or MAIT cells may represent greater than 2% (e.g., between 2% and 100%, between 10% and 95%, between 20% and 90%, between 30% and 80%, between 40% and 70%, e.g., greater than 5%, greater than 10%, greater than 15%, greater than 20%, greater than 30%, greater than 40%, greater than 50%, greater than 60%, greater than 70%, greater than 80%, greater than 90%, greater than 95%, 96%, 97%, 98%, or 99%) of the total number of cells in the isolated cell population. In some embodiments, the isolated cell population includes at least ten engineered γδ T cells, NK cells, NK-like T cells, innate lymphoid cells, or MAIT cells of any one of the preceding embodiment.

In another aspect, the invention includes a γδ T cell, NK cell, NK-like T cell, innate lymphoid cell, or MAIT cell including a heterologous polynucleotide. The heterologous polynucleotide may encode a heterologous targeting construct including an extracellular antigen-binding domain and a transmembrane domain operatively linked to the antigen-binding domain, wherein the heterologous targeting construct does not directly activate the engineered γδ T cell, NK cell, NK-like T cell, innate lymphoid cell, or MAIT cell.

In yet another aspect, the invention features a γδ T cell, NK cell, NK-like T cell, innate lymphoid cell, or MAIT cell that includes a heterologous polynucleotide encoding a targeting construct that includes an antigen-binding domain and a terminal transmembrane domain.

An engineered γδ T cell, NK cell, NK-like T cell, innate lymphoid cell, or MAIT cell; isolated engineered γδ T cell population, NK cell, NK-like T cell, innate lymphoid cell, or MAIT cell population; or the γδ T cell, NK cell, NK-like T cell, innate lymphoid cell, or MAIT cell including a heterologous polynucleotide of any of the preceding embodiments can be used in a method of treating a subject by adoptive cell therapy (e.g., for use in a method of treating a subject by adoptive cell therapy).

In another aspect, the invention features a method of treating a subject by adoptive cell therapy (e.g., adoptive T cell therapy) that includes administering a therapeutically effective amount of the engineered cells, isolated cell population, or cells of any of the preceding embodiments to a subject in need thereof.

In another aspect, the invention provides the engineered cells, isolated cell population, or cells of any of the preceding embodiments for use in a method of treating a subject by adoptive cell therapy (e.g., adoptive T cell therapy), wherein the method includes administering a therapeutically effective amount of the engineered cells, isolated cell population, or cells of any of the preceding embodiments to a subject in need thereof.

In some embodiments of any of the preceding aspects, the subject is a human. For example, the subject may be a human cancer patient (e.g., a human cancer patient being treated for a solid tumor). Alternatively, the human patient may be a human patient being treated for a viral infection.

## BRIEF DESCRIPTION OF THE DRAWINGS

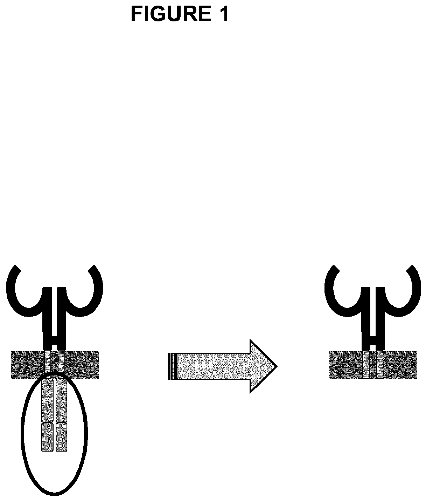

FIG. 1 is a schematic drawing showing a classical chimeric antigen receptor (CAR) versus one embodiment of a heterologous targeting construct, which does not include an intracellular domain.

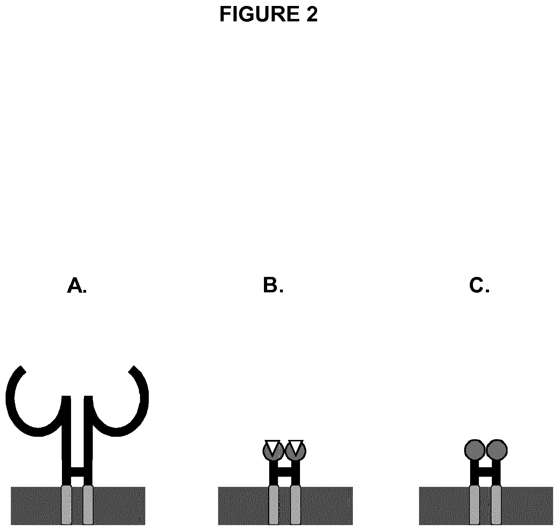

FIGS. 2A-2C are a series of schematic drawings showing how the heterologous targeting construct can be modified with various extracellular domains tailored to the desired target. FIG. 2A shows a generalized extracellular domain which can be, for example, a B-cell receptor, an antibody scaffold or mimetic, an scFv, a mAb, a Fab, or a T cell receptor. FIG. 2B shows an extracellular domain that is a ligand-specific receptor. FIG. 2C shows an extracellular domain that is a receptor-specific ligand.

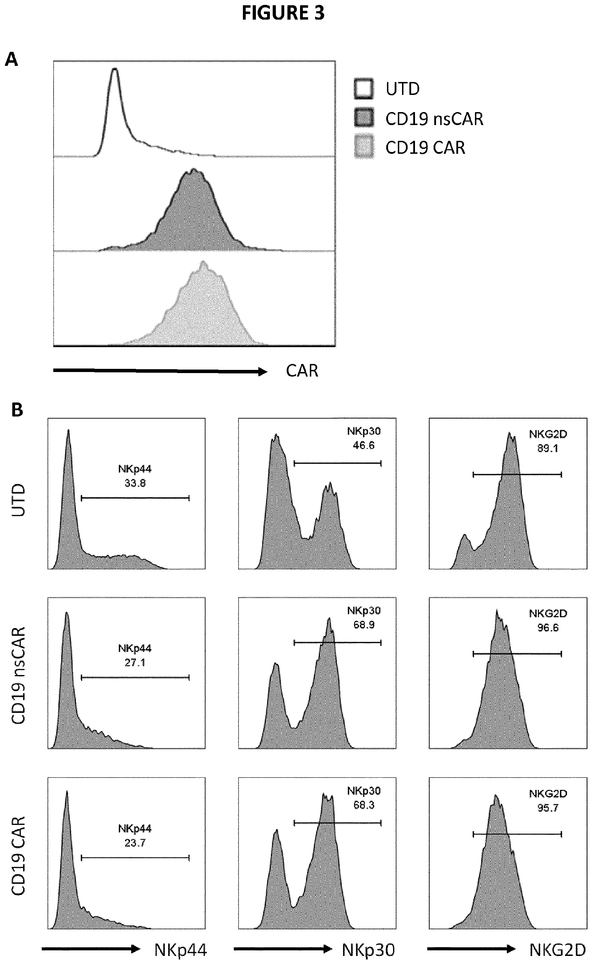

FIGS. 3A and 3B are flow cytometry histograms. FIG. 3A shows the expression of an anti-CD19 targetting construct without an intracellular domain ("nonsignalling or nsCAR") and a full length anti-CD19 CAR on transduced Vδ1 cells.

FIG. 3B shows expression of NCR (natural cytotoxicity receptors) NKp30 (left-hand column), NKp44 (middle column), and NKG2D (right-hand-column) on Vδ1 cells that are untransduced (UTD; top row), transduced with nonsignaling CD19 CAR (middle row), and transduced with CD19 CAR (bottom row).

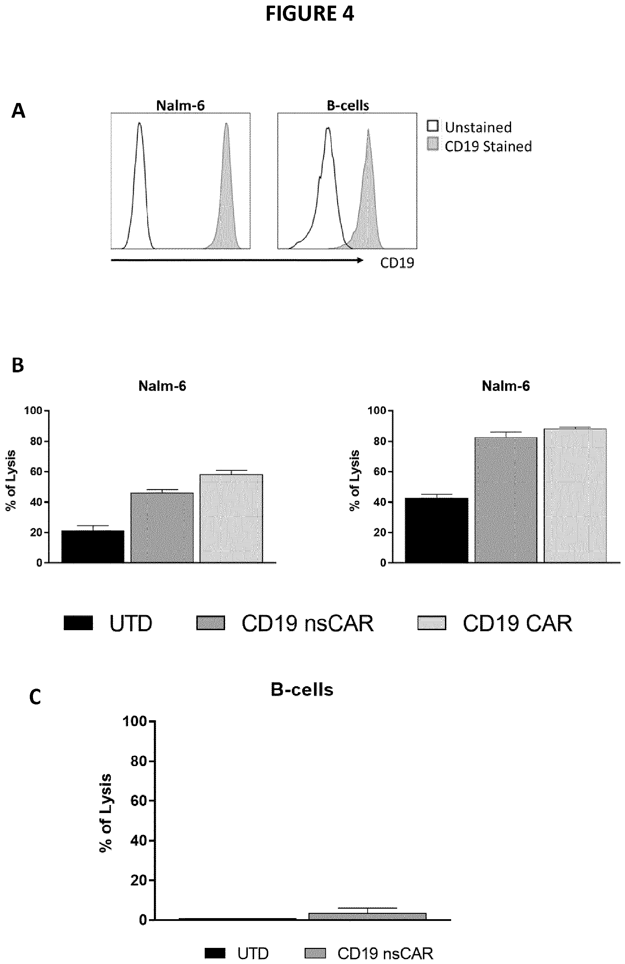

FIGS. 4A-4C are graphs showing CD19 expression on Nalm-6 and B-cells (FIG. 4A) and results from a 16-hour killing assay at 1:1 effector to target ratio (FIGS. 4B and 4C). FIG. 4B shows killing of CD19+ Nalm-6 cells, and FIG. 4C shows killing of primary B-ALL cells. Two independent donors and experiments are shown.

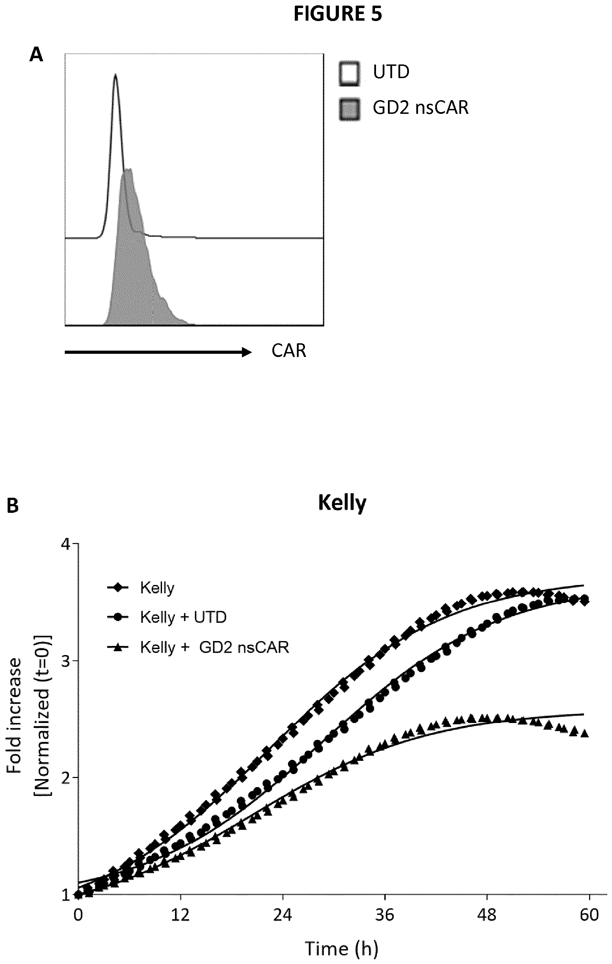

FIGS. 5A and 5B are graphs showing anti-GD2 nonsignalling CAR expression on Vδ1 cells (FIG. 5A) and a 60-hour time course of Kelly cell line growth alone or in the presence of Vδ1 cells (FIG. 5B). Data are expressed as the change in number in green object count per image normalised to the number in green object count per image at time zero. Each data point represents triplicate wells.

## DETAILED DESCRIPTION

Provided herein are compositions of engineered lymphocytes (e.g., lymphocytes having innate-like effector functions, such as γδ T cells, NK cells, NK-like T cells, lymphoid cells, or mucosal-associated invariant T cells) expressing a heterologous targeting construct. The heterologous targeting construct includes an extracellular antigen-binding domain and a transmembrane domain operatively linked to the antigen-binding domain (e.g., directly linked or linked through a stalk domain). These engineered lymphocytes (e.g., γδ T cells) may be used for treatment of diseases, such as cancers or viral infections. Because the heterologous constructs of the present invention lack a functional intracellular domain capable of propagating T cell activation, they rely on endogenous MHC-independent activation pathways characteristic of γδ T cells, which are lacking in αβ T cells. Thus, the heterologous constructs described herein are designed to be expressed on the surface of lymphocytes, e.g., γδ T cells (e.g., Vδ1 cells, Vδ2 cells Vδ3 cells, Vδ5 cells, and Vδ8 cells).

**Definitions**

It is to be understood that aspects and embodiments of the invention described herein include "comprising," "consisting," and "consisting essentially of" aspects and embodiments. As used herein, the singular form "a," "an," and "the" includes plural references unless indicated otherwise.

The term "about" as used herein refers to the usual error range for the respective value readily known to the skilled person in this technical field. Reference to "about" a value or parameter herein includes (and describes) embodiments that are directed to that value or parameter *per se.* In some instances, "about" encompass variations of +20%, in some instances +10%, in some instances +5%, in some instances +1%, or in some instances +0.1% from the specified value, as such variations are appropriate to perform the disclosed methods.

As used herein, the terms "substantial" and "substantially" refer to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena. When describing a physical scenario, such as receptor/ligand interaction or cell/cell contact, the scenario is substantial if its functional result is detectable by conventional means available to the person performing the method. For example, "substantial TCR activation" refers to a detectable level of TCR activation among a population of cells (e.g., a statistically significant degree of TCR activation). In some embodiments, a TCR is substantially activated upon exposure to up to 0.1 %, up to 0.5%, up to 1 %, up to 5%, up to 10%, up to 20%, up to 30%, or up to 40% of the $EC_{50}$ of the TCR pathway agonist (e.g., an antibody, e.g., anti-CD3, or a lectin) on the respective cell population.

As used herein, a "heterologous targeting construct" refers to a protein or set of proteins (e.g., two or more proteins that dimerize to form a functional quaternary protein) that resides on a host cell (i.e., an engineered cell) and binds a target molecule present on another cell, and which is not naturally expressed by the cell on which it resides. A heterologous targeting construct may be encoded by a polynucleotide expressed within the engineered cell.

As used herein, to "activate" a T cell means to initiate or amplify the T cell receptor (TCR) pathway by propagating signal 1 activation or signal 2 activation. For example, a chimeric antigen receptor having a functional signal 1 T cell activating domain (e.g., CD3ζ) or co-stimulatory domain (e.g., CD28, 4-1BB, etc.) may "activate" its host T cell by clustering in response to antigen-binding. A heterologous targeting construct lacking a functional intracellular domain may have no means of propagating signal 1 activation or signal 2 activation and therefore cannot activate the TCR pathway. A heterologous targeting construct lacking a functional intracellular domain may be capable of "activating" the T cell on which it is expressed if its transmembrane domain propagates co-stimulation, e.g., upon association of an NKG2D transmembrane domain with endogenous DAP10 or DAP12. In alternative embodiments, the invention features heterologous targeting constructs having transmembrane domains that are nonfunctional, and the heterologous targeting domain does not activate the T cell on which it is expressed.

Activation of the "T cell receptor (TCR) pathway" refers to the induction of proliferation or other consequences of activation of T cells through TCR signaling. The TCR signaling pathway involves signal 1 activation, e.g., sequential activation of the Src-related protein tyrosine kinases (PTKs), Lck and Fyn, and zeta-chain (TCR) associated protein kinase of 70 kDA (ZAP70). These PTKs lead to phosphorylation of polypeptides including linker activator for T cells (LAT), which leads to downstream stimulation through extracellular signal regulated kinase (ERK), c-Jun N-terminal kinase (JNK), and nuclear factor of activated T cells (NFAT). Signal 2 (i.e., co-stimulation), for example through CD28, CD45, DAP10, or DAP12 can enhance phosphorylation and enhance TCR activation. Thus, any molecule that targets a part of the TCR or co-stimulatory pathway can directly activate T cell signaling. Surface-bound molecules that simply bring a T cell into contact with a target cell may facilitate other molecules to directly trigger T cell activation (e.g., a heterologous targeting construct) but these targeting molecules do not directly activate the TCR pathway.

TCR pathway agonists include antibodies (e.g., monoclonal antibodies, e.g., anti-TCR Vδ1, anti-TCR δTCS-1, anti-TCR PAN γδ, and anti-CD3), lectins (e.g., plant lectins, e.g., *Concanavalin A,* lectins from *Phaseolus vulgaris* (PHA-P), *Phytolacca Americana, Triticum vulgaris, Lens culinaris, Glycine max, Maackia amurensis, Pisum sativum,* and *Sambucus nigra),* synthetic phosphoantigens (e.g., BrHPP (bromohydrin pyrophosphate), 2M3B1PP (2-methyl-3-butenyl-1-pyrophosphate), HMBPP ((E)-4-Hydroxy-3-methyl-but-2-enyl pyrophosphate), or IPP (isopentenyl pyrophosphate)), and N-bisphosphonates (e.g., zoledronate). TCR pathway agonists include co-receptor agonists, including antibodies (e.g., monoclonal antibodies, e.g., anti CD2, anti-CD6, anti-CD9, anti-CD28, anti-CD43, anti-CD94, anti-CD160, anti-SLAM, anti-NKG2D, anti-2B4, anti-HLA-A, anti-HLA-b, anti-HLA-C, and anti-ICAM-3) and proteins (e.g., recombinant proteins, e.g., recombinant human proteins, e.g., CD7L, CD26, CD27L, CD30L, CD40L, OX40L, 4-1BBL, ICAM-1, fibronectin, hydrocortisone, and variants thereof, e.g., Fc-fusion proteins, e.g., CD27L-Fc). TCR pathway agonists may be soluble or membrane bound and may, for example, be presented on cells, such as artificial antigen presenting cells (aAPCs), as is the case for MHC or HLA complexes. Suitable aAPCs for activating T cell signaling are known in the art. Suitable methods of activating T cells by exogenously adding TCR pathway agonists are well known in the art and summarized in Figure 1 of Deniger, et al. (Deniger, et al. Frontiers in Immunology. 2014. 5(636):1-10).

"Exogenous TCR pathway agonists" refer to TCR pathway agonists that do not originate from the non-haematopoietic tissue or donor thereof (i.e., they are exogenously added). Thus, it will be understood that in some embodiments of the invention, a TCR pathway agonist may be present in the culture as residual material from the non-haematopoietic tissue (e.g., soluble fibronectin or cell-bound ICAM-1). In some embodiments, a residual TCR pathway agonist is of a negligible concentration and does not substantially activate the T cells.

For a domain of a protein, such as a heterologous targeting construct, to be "operatively linked" to another domain herein means to be reside on the same protein as the other domain, either directly adjacent to the other domain or separated by one or more amino acids or domains. For example, in a heterologous targeting construct having an N-terminal antigen-binding domain, an intermediate stalk domain, and a C-terminal transmembrane domain, the antigen-binding domain and the transmembrane domain are said to be operatively linked. In a heterologous targeting construct having an N-terminal antigen-binding domain immediately adjacent to a C-terminal transmembrane domain, the antigen-binding domain and the transmembrane domain are also said to be operatively linked but, more specifically, are directly linked.

As used herein, the term "antibody scaffold" refers to a non-native antigen-binding protein, peptide, or antibody fragment. Antibody scaffolds include adnectins, affibodies, affilins, anticalins, atrimers, avimers, bicyclic peptides, centyrins, cys-knots, DARPins, fynomers, Kunitz domains, Obodies, and Tn3s. Antibody scaffolds are known in the art and described, for example, in Vazquz-Lombardi et al, Drug Discovery Today, 2015, 20(10): 1271-83, which is incorporated herein by reference in its entirety.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

As used herein, the term "cytotoxicity" refers to the ability of immune cells (e.g., $\gamma\delta$ T cells) to kill other cells (e.g., target cells). Immune cells with cytotoxic functions release toxic proteins (e.g., perforin and granzymes) capable of killing nearby cells.

As used herein, the term "degranulation" refers to a cellular process in which molecules, including antimicrobial and cytotoxic molecules, are released from intracellular secretory vesicles called granules. Degranulation is part of the immune response to pathogens and invading microorganisms by immune cells such as cytotoxic T cells. The molecules released during degranulation vary by cell type and can include molecules designed to kill the invading pathogens and microorganisms or to promote an immune response, such as inflammation.

As used herein, the term "innate lymphoid cell" refers to an innate-like lymphocyte lacking rearranged antigen receptors such as those expressed by T and B cells. Innate lymphoid cells include NK cells, type 1 innate lymphoid cells (ILC1), intra-ILC1 cells, type 2 innate lymphoid cells (ILC2), type 3 innate lymphoid cells (ILC3), etc.

As used herein, the terms "mucosal-associated invariant T cell" and "MAIT cell" refer to an innate-like T cell that expresses an invariant T cell receptor $\alpha$ (TCR$\alpha$) chain and a diverse TCR$\beta$ chain and can recognize a distinct set of molecules in the context of an evolutionarily conserved major histocompatibility complex-related molecule 1 (MR1).

As used herein, the term "NK cell" refers to a natural killer cell, an innate-like lymphocyte that does not express a TCR or CD3 and is positive for expression of CD56 and CD161. NK cells can also express natural cytotoxicity receptors, such as NKp44 and NKp46.

As used herein, the term "NK-like T cell" refers to natural killer-like T cells, or natural killer T cells (NKT cells), which are innate-like lymphocytes that express that share functional and structural characteristics with both T cells and NK cells, i.e., they express a TCR (e.g., $\alpha\beta$ TCR), CD3, and CD56. NK-like T cells recognize and react against glycolipids in the context of the MHC class-I-like glycoprotein, CD1d, and can produce IFN-$\gamma$ and IL-4 upon activation.

As used herein, "non-haematopoietic cells" include stromal cells and epithelial cells. Stromal cells are non-haematopoietic connective tissue cells of any organ and support the function of the parenchymal cells of that organ. Examples of stromal cells include fibroblasts, pericytes, mesenchymal cells, keratinocytes, endothelial cells, and non-hematological tumor cells. Epithelial cells are non-haematopoietic cells that line the cavities and surfaces of blood vessels and organs throughout the body. They are normally squamous, columnar, or cuboidal in shape and can be arranged as a single layer of cells, or as layers of two or more cells.

As used herein, "non-haematopoietic tissue-resident γδ T cells," "non-haematopoietic tissue-derived," and "non-haematopoietic tissue-native γδ T cells" refer to γδ T cells that were present in a non-haematopoietic tissue at the time the tissue is explanted. Non-haematopoietic tissue-resident γδ T cells may be obtained from any suitable human or non-human animal non-haematopoietic tissue. Non-haematopoietic tissue is a tissue other than blood or bone marrow. In some embodiments, the γδ T cells are not obtained from particular types of samples of biological fluids, such as blood or synovial fluid. Examples of such suitable human or non-human animal non-haematopoietic tissues include skin or a portion thereof (e.g., dermis or epidermis), the gastrointestinal tract (e.g. gastrointestinal epithelium, colon, small intestine, stomach, appendix, cecum, or rectum), mammary gland tissue, lung (preferably wherein the tissue is not obtained by bronchoalveolar lavage), prostate, liver, and pancreas. In some embodiments, non-haematopoietic tissue-resident γδ T cells can be derived from a lymphoid tissue, such as thymus, spleen, or tonsil. The γδ T cells may also be resident in human cancer tissues, e.g. breast and prostate. In some embodiments, the γδ T cells are not obtained from human cancer tissue. Non-haematopoietic tissue samples may be obtained by standard techniques e.g., by explant (e.g., biopsy). Non-haematopoietic tissue-resident γδ T cells include e.g., Vδ1 T cells, double negative (DN) T cells, Vδ2 T cells, Vδ3 T cells, and Vδ5 T cells.

Any one or more of the above factors may be included in an expansion protocol in an amount effective to produce an expanded population of lymphocytes (e.g., γδ T cells), which may be transfected with a nucleic acid encoding a heterologous targeting construct of the invention. As used herein, the phrase "in an amount effective to" refers to an amount that induces a detectable result (e.g., a number of cells having a statistically significant increased number relative to its starting population, e.g., at a $p < 0.05$). In instances in which multiple factors are present at once, an effective amount refers to the composite effect of all factors (e.g., the composite effect of IL-2 and IL-15, or the composite effect of IL-2, IL-4, IL-15, and IL-21).

As used herein, an "expanded population of γδ cells" refers to a population of haematopoietic or non-haematopoietic cells including γδ T cells that has been cultured in a condition and for a duration that has induced the expansion of γδ cells, i.e., increased γδ cell number. Likewise, an "expanded population of Vδ1 T cells," as used herein, refers to a population of haematopoietic or non-haematopoietic cells including Vδ1 T cells that has been cultured in a condition and for a duration that has induced the expansion of Vδ1 T cells, i.e., increased Vδ1 cell number.

As used herein, a "feeder cell" refers to any exogenous cell added to a culture to provide cell-to-cell surface contact to the non-haematopoietic tissue-derived cells. Feeder cells can be primary cells (e.g., derived from a tissue) or a derived from a cell line. Feeder cells can be live or irradiated, and include tumor cells, fibroblasts, B cells, and other antigen presenting cells.

The term "marker" herein to refers to a DNA, RNA, protein, carbohydrate, glycolipid, or cell-based molecular marker, the expression or presence of which in a patient's sample can be detected by standard methods (or methods disclosed herein).

A cell or population of cells that "expresses" a marker of interest is one in which mRNA encoding the protein, or the protein itself, including fragments thereof, is determined to be present in the cell or the population. Expression of a marker can be detected by various means. For example, in some embodiments, expression of a marker refers to a surface density of the marker on a cell. Mean fluorescence intensity (MFI), for example, as used as a readout of flow cytometry, is representative of the density of a marker on a population of cells. A person of skill in the art will understand that MFI values are dependent on staining parameters (e.g., concentration, duration, and temperature) and fluorochrome composition. However, MFI can be quantitative when considered in the context of appropriate controls. For instance, a population of cells can be said to express a marker if the MFI of an antibody to that marker is significantly higher than the MFI of an appropriate isotype control antibody on the same population of cells, stained under equivalent conditions. Additionally or alternatively, a population of cells can be said to express a marker on a cell-by-cell basis using a positive and negative gate according to conventional flow cytometry analytical methods (e.g., by setting the gate according to isotype or "fluorescence-minus-one" (FMO) controls). By this metric, a population can be said to "express" a marker if the number of cells detected positive for the marker is significantly higher than background (e.g., by gating on an isotype control).

As used herein, when a population's expression is stated as a percentage of positive cells and that percentage is compared to a corresponding percentage of positive cells of a reference population, the percentage difference is a percentage of the parent population of each respective population. For example, if a marker is expressed on 10% of the cells of population A, and the same marker is expressed on 1 % of the cells of population B, then population A is said to have a 9% greater frequency of marker-positive cells than population B (i.e., 10%-1 %, not 10%÷1 %). When a frequency is multiplied through by the number of cells in the parent population, the difference in absolute number of cells is calculated. In the example given above, if there are 100 cells in population A, and 10 cells in population B, then population A has 100-fold the number of cells relative to population B, i.e., (10% x 100) ÷ (1 % x 10).

An expression level of a marker may be a nucleic acid expression level (e.g., a DNA expression level or an RNA expression level, e.g., an mRNA expression level). Any suitable method of determining a nucleic acid expression level may be used. In some embodiments, the nucleic acid expression level is determined using qPCR, rtPCR, RNA-seq, multiplex qPCR or RT-qPCR, microarray analysis, serial analysis of gene expression (SAGE), MassARRAY technique, in situ hybridization (e.g., FISH), or combinations thereof.

As used herein, a "reference population" of cells refers to a population of cells corresponding to the cells of interest, against which a phenotype of the cells of interest are measured. For example, a level of expression of a marker on a separated population of non-haematopoietic tissue-derived $\gamma\delta$ cells may be compared to the level of expression of the same marker on a haematopoietic tissue-derived $\gamma\delta$ T cell (e.g., a blood-resident $\gamma\delta$ cell, e.g., a blood-resident $\gamma\delta$ cell derived from the same donor or a different donor) or a non-haematopoietic tissue-derived $\gamma\delta$ T cell expanded under different conditions (e.g., in the presence of substantial TCR activation, in the presence of an exogenous TCR activation agent (e.g., anti-CD3), or in substantial contact with stromal cells (e.g., fibroblasts)). A population may also be compared to itself at an earlier state. For example, a reference population can be a separated cell population prior to its expansion. In this case, the expanded population is compared to its own composition prior to the expansion step, i.e., its past composition, in this case, is the reference population.

"Cancer" refers to the abnormal proliferation of malignant cancer cells and includes hematopoietic cancer (e.g., a hematological malignancy such as a leukemia, such as acute myeloid leukemia (AML), chronic myeloid leukemia (CML), chronic eosinophilic leukemia (CEL), myelodysplastic syndrome (MDS), acute lymphoblastic leukemia (ALL), and chronic lymphocytic leukemia (CLL), lymphomas, such as Hodgkin lymphoma, non-Hodgkin lymphoma (NHL) and multiple myeloma (MM)), and solid cancers such as sarcomas (e.g., soft tissue sarcoma, uterine sarcoma), skin cancer, melanoma (e.g., malignant melanoma), bladder cancer, brain cancer, breast cancer, uterus cancer, ovary cancer, prostate cancer, lung cancer, colorectal cancer (e.g., colorectal adenocarcinoma), cervical cancer, liver cancer (i.e., hepatic cancer), head and neck cancer (e.g., head and neck squamous cell carcinoma), esophageal cancer, pancreas cancer, renal cancer (e.g., renal cell carcinoma), adrenal cancer, stomach cancer, gastric cancer (e.g., gastric adenocarcinoma), testicular cancer, cancer of the gall bladder and biliary tracts, thyroid cancer, thymus cancer, cancer of bone, cerebral cancer, biliary cancer, bladder cancer, bone and soft tissue carcinoma, brain tumour, cervical cancer, colon cancer, desmoid tumour, embryonal cancer, endometrial cancer, oesophageal cancer, gastric adenocarcinoma, glioblastoma multiforme, gynaecological tumour, osteosarcoma, ovarian cancer, pancreatic cancer, pancreatic ductal adenocarcinoma, primary astrocytic tumor, primary thyroid cancer, rhabdomyosarcoma, skin cancer, testicular germ-cell tumor, urothelial cancer, and uterine cancer. Cancer cells within cancer patient may be immunologically distinct from normal somatic cells in the individual (e.g., the cancerous tumor may be immunogenic). For example, the cancer cells may be capable of eliciting a systemic immune response in the cancer patient against one or more antigens expressed by the cancer cells. The antigens that elicit the immune response may be tumor antigens or may be shared by normal cells. A patient with cancer may display at least one identifiable sign, symptom, or laboratory finding that is sufficient to make a diagnosis of cancer in accordance with clinical standards known in the art. Examples of such clinical standards can be found in textbooks of medicine such as Harrison's Principles of Internal Medicine (Longo DL, Fauci AS, Kasper DL, Hauser SL, Jameson J, Loscalzo J. eds. 18e. New York, NY: McGraw-Hill; 2012). In some instances, a diagnosis of a cancer in an individual may include identification of a particular cell type (e.g. a cancer cell) in a sample of a body fluid or tissue obtained from the individual.

As used herein, a "solid tumor" is any cancer of body tissue other than blood, bone marrow, or the lymphatic system. Solid tumors can be further divided into those of epithelial cell origin and those of non-epithelial cell origin. Examples of epithelial cell solid tumors include tumors of the gastrointestinal tract, colon, breast, prostate, lung, kidney, liver, pancreas, ovary, head and neck, oral cavity, stomach, duodenum, small intestine, large intestine, anus, gall bladder, labium, nasopharynx, skin, uterus, male genital organ, urinary organs, bladder, and skin. Solid tumors of non-epithelial origin include sarcomas, brain tumors, and bone tumors.

A patient, subject, or individual suitable for treatment as described above may be a mammal, such as a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. a marmoset or baboon), an ape (e.g. a gorilla, chimpanzee, orangutan or gibbon), or a human.

In some embodiments, the patient, subject, or individual is a human. In other preferred embodiments, non-human mammals, especially mammals that are conventionally used as models for demonstrating therapeutic efficacy in humans (e.g. murine, primate, porcine, canine, or rabbit) may be employed.

As used herein, "treatment" (and grammatical variations thereof such as "treat" or "treating") refers to clinical intervention, whether of a human or an animal (e.g. in veterinary applications), in which some desired therapeutic effect is achieved, for example, the inhibition or delay of the progress of the condition, and includes a reduction in the rate of progress, a halt in the rate of progress, amelioration of the condition, cure or remission (whether partial or total) of the condition, preventing, delaying, abating or arresting one or more symptoms and/or signs of the condition or prolonging survival of a subject or patient beyond that expected in the absence of treatment.

Treatment as a prophylactic measure (i.e. prophylaxis) is also included. For example, a patient, subject, or individual susceptible to or at risk of the occurrence or re-occurrence of cancer may be treated as described herein. Such treatment may prevent or delay the occurrence or re-occurrence of cancer in the patient, subject, or individual.

In particular, treatment may include inhibiting cancer growth, including complete cancer remission, and/or inhibiting cancer metastasis. Cancer growth generally refers to any one of a number of indices that indicate change within the cancer to a more developed form. Thus, indices for measuring an inhibition of cancer growth include a decrease in cancer cell survival, a decrease in tumor volume or morphology (for example, as determined using computed tomographic (CT), sonography, or other imaging method), a delayed tumor growth, a destruction of tumor vasculature, improved performance in delayed hypersensitivity skin test, an increase in the activity of cytolytic T-lymphocytes, and a decrease in levels of tumor-specific antigens. Reducing immune suppression in cancerous tumors in an individual may improve the capacity of the individual to resist cancer growth, in particular growth of a cancer already present the subject and/or decrease the propensity for cancer growth in the individual.

In some embodiments, expanded γδ T cells (e.g., non-haematopoietic tissue-derived γδ T cells, e.g., non-haematopoietic tissue-derived Vδ1 T cells) are administered to delay development of a disease or to slow the progression of a disease or disorder.

As used herein, "administering" is meant a method of giving a dosage of a therapy (e.g., an adoptive T cell therapy including, e.g., non-haematopoietic tissue-derived γδ T cells) or a composition (e.g., a pharmaceutical composition, e.g., a pharmaceutical composition including non-haematopoietic tissue-derived γδ T cells) to a patient. The compositions utilized in the methods described herein can be administered, for example, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subcutaneously, subconjunctivally, intravesicularly, mucosally, intrapericardially, intraumbilically, intraocularly, intraorbitally, intravitreally (e.g., by intravitreal injection), by eye drop, orally, topically, transdermally, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the therapeutic agent or composition being administered and the severity of the condition, disease, or disorder being treated).

A "therapeutically effective amount" refers to an amount of a therapeutic agent to treat or prevent a disease or disorder in a mammal. In the case of cancers, the therapeutically effective amount of the therapeutic agent (e.g., a non-haematopoietic tissue-derived γδ T) may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), response rates (e.g., complete response (CR) and partial response (PR)), duration of response, and/or quality of life.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s). For example, in some embodiments, a non-haematopoietic tissue-derived γδ T cell and IL-2 may be administered concurrently.

The term "pharmaceutical composition" refers to a preparation which is in such form as to permit the biological activity of one or more active ingredients contained therein to be effective, and which contains no additional components which are unacceptably toxic to a patient to which the formulation would be administered.

As used herein, a "terminal transmembrane domain" refers to a transmembrane domain having an unlinked terminal end (e.g., a C-terminus that is not linked to a peptide or protein). Thus, a terminal transmembrane domain is not linked to an intracellular domain, such as an intracellular signaling domain. In some embodiments, a terminal transmembrane domain does not participate in an intracellular signaling pathway (e.g., a T cell signaling pathway, such as signal 1 activation or signal 2 co-stimulation).

As used herein, the term "Chimeric Antigen Receptor" or alternatively a "CAR" refers to a recombinant polypeptide construct including an extracellular antigen binding domain, a transmembrane domain, and an intracellular domain that propagates an activation signal that activates the cell. In some embodiments, the CAR includes an optional leader sequence at the N-terminus of the CAR fusion protein.

In the event of any conflicts or inconsistencies between the definitions set forth herein and the definitions provided in any of the references incorporated herein by reference, the definitions set forth herein shall control.

### γδ T cells and other Innate-like Lymphocytes Expressing Heterologous Targeting Constructs

Lymphocytes such as γδ T cells and other innate-like lymphocytes (e.g., innate lymphoid cells, such as NK cells and NK-like T cells, and mucosal-associated invariant T (MAIT) cells) are attractive vehicles for heterologous targeting constructs described herein, as they can be transduced with heterologous targeting constructs while retaining their innate-like capabilities of recognizing pathogenic cells, such as cancer cells and infected cells. Transduction can be performed using any suitable method known in the art or described herein, such as by electroporation, gene editing (e.g., by clustered regularly interspaced short palindromic repeats (CRISPR), zinc finger nuclease (ZFN) transfection), transposon-delivered, etc. Furthermore, the lack of MHC-dependent antigen recognition, for example, by $\gamma\delta$ T cells, reduces the potential for graft-versus-host disease and permits them to target tumors expressing low levels of MHC. Likewise, the non-reliance of $\gamma\delta$ T cells upon conventional signal 2 co-stimulation, for example, via engagement of CD28, enhances the targeting of tumors expressing low levels of ligands for co-stimulatory receptors.

In one aspect, the invention provides $\gamma\delta$ T cells, NK cells, NK-like T cells, innate lymphoid cells, and MAIT cells and cell populations thereof, expressing a heterologous targeting construct on their surface. Such $\gamma\delta$ T cells, NK cells, NK-like T cells, innate lymphoid cells, and MAIT cells engineered to express a heterologous targeting construct can be utilized to target a desired antigen with through an antigen-binding domain on the heterologous construct. Because $\gamma\delta$ T cells do not rely on MHC receptors to respond to foreign pathogens, the heterologous targeting construct does not require an intracellular domain to induce cytolysis or cytotoxicity, in contrast to conventional chimeric antigen receptor (CAR) systems used as part of conventional (e.g., $\alpha\beta$) T cell adoptive immunotherapy regimens. Instead, $\gamma\delta$ T cells elicit an intrinsic target-specific cytolysis, and this response can be further enhanced by improving and increasing the contact time with the target cell (e.g., a tumor, e.g., a solid tumor) by using a heterologous construct. The $\gamma\delta$ T cell engineered with a heterologous construct can bind a target antigen, such as a tumor-associated antigen, and induce cytotoxicity and/or cytolysis. This cytotoxicity can be mediated through endogenous expression of activating receptors such as NKG2D, NKp30, NKp44, NKp46, and/or DNAM1.

The heterologous targeting construct may feature an extracellular antigen-biding domain and a transmembrane domain operatively linked to the antigen-binding domain. A stalk domain may further be included as part of the heterologous targeting construct to link the antigen-binding domain to the transmembrane domain. In some embodiments, the heterologous targeting construct provided herein lacks an intracellular domain (FIG. 1) and also lacks the capacity to activate TCR signaling (e.g., through signal 1 activation and/or signal 2 activation (i.e., co-stimulation).

In some embodiments, cytolysis is characterized by degranulation (e.g., CD107 degranulation) of the $\gamma\delta$ T cell, granzyme release by the $\gamma\delta$ T cell, perforin release $\gamma\delta$ T cell, target cell killing, proliferation of the $\gamma\delta$ T cell, or cytokine production by the $\gamma\delta$ T cell. One of skill in the art will recognize that various assays that measure these properties or activities can be used to assess the efficacy of a engineered T cell, e.g., in treating cancer.

In general, degranulation is a pre-requisite for cytolysis. Degranulating cells can be identified, e.g., by the surface expression of LAMP-1 (lysosomal associated membrane protein 1, also known as CD107). CD107 is expressed transiently on the surface and rapidly internalizes after degranulation. In a non-activated state, CD107a resides in the cytoplasm in the cytolytic granule membrane. Upregulation can be measured (e.g., by FACS) by staining CD107 in the presence of monensin to prevent acidification of antibody labelled internalized CD107a-containing vesicles.

Perforin and granzyme assays can also be measured by FACS, according to methods known in the art. Cytotoxic $\gamma\delta$ T cells kill their target by granule or receptor mediated mechanisms. Cytotoxic granules are secretory lysosomes pre-formed in the cytoplasm containing lytic proteins (perforin and granzymes). Upon target cell recognition, the lytic proteins are secreted by exocytosis. Upon target cell recognition, the decrease of intracellular granzyme and/or perforin level can thus be measured by FACS.

Cell-killing assays may be used to monitor the effect of a $\gamma\delta$ T cell expressing a heterologous targeting construct. A kinetic target cell lysis assay may be used to track the percent of killing over time at various effector to target ratios. An endpoint target cell lysis assay (e.g., luciferase assay) may be used to track the percent of killing at a specific endpoint time at various effector to target ratios. Immunological synapse formation (e.g., observed by live cell imaging) may be used to measure binding kinetics, target recognition (e.g., Ca flux in effector cells), lethal hit (e.g., as measured by propidium iodide blush in target cells), or target cell rounding.

In some embodiments, the binding of the antigen-binding domain to a target antigen expressed on a healthy cell does not substantially trigger cytolysis in the engineered $\gamma\delta$ T cell. In some embodiments, binding of the antigen-binding domain to a target antigen expressed on a tumor cell or an infected cell substantially triggers cytolysis in the engineered $\gamma\delta$ T cell.

In one aspect, the invention provides a cell (e.g., $\gamma\delta$ T cell, NK cell, NK-like T cell, innate lymphoid cell, or MAIT cell) engineered to express a heterologous targeting construct, wherein the engineered cell exhibits an antitumor property. In one aspect, a cell is transfected (e.g., by nucleofection, electroporation, etc.) with the heterologous targeting construct and the heterologous targeting construct is expressed on the cell surface. In some embodiments, the cell (e.g., $\gamma\delta$ T cell, NK cell, NK-like T cell, innate lymphoid cell, or MAIT cell) is transduced with a viral vector encoding a heterologous targeting construct. In some embodiments, the viral vector is a retroviral vector. In some embodiments, the viral vector is a lentiviral vector. In some such embodiments, the cell may stably express the heterologous targeting construct. In another embodiment, the cell (e.g., $\gamma\delta$ T cell, NK cell, NK-like T cell, innate lymphoid cell, or MAIT cell) is transfected (e.g., by nucleofection, electroporation, etc.) with a nucleic acid, e.g., mRNA, cDNA, DNA, encoding a heterologous targeting construct. In some embodiments, the cell may transiently express the heterologous targeting construct.

In one aspect, the invention features a cell population (e.g., an isolated cell population) of engineered $\gamma\delta$ T cells (e.g., at least 10, $10^2$, $10^3$, $10^4$, $10^5$, $10^6$, $10^7$, $10^8$, $10^9$, $10^{10}$, $10^{11}$, $10^{12}$, or $10^{13}$ cells), where at least 10% (e.g., 20%, 30%, 40%, 50%, 60%, 70%, 80%, 90%, 95%, 97%, 99%, or substantially all) of the cell population are of engineered $\gamma\delta$ T cells expressing a heterologous targeting construct.

Alternatively, the invention features a cell population (e.g., an isolated cell population) of engineered NK cells or NK-like T cells (e.g., at least 10, $10^2$, $10^3$, $10^4$, $10^5$, $10^6$, $10^7$, $10^8$, $10^9$, $10^{10}$, $10^{11}$, $10^{12}$, or $10^{13}$ NK cells or NK-like T cells), where at least 10% (e.g., 20%, 30%, 40%, 50%, 60%, 70%, 80%, 90%, 95%, 97%, 99%, or substantially all) of the cell population is engineered to express a heterologous targeting construct.

Alternatively, the invention features a cell population (e.g., an isolated cell population) of engineered innate lymphoid cells (e.g., at least 10, $10^2$, $10^3$, $10^4$, $10^5$, $10^6$, $10^7$, $10^8$, $10^9$, $10^{10}$, $10^{11}$, $10^{12}$, or $10^{13}$ NK cells or NK-like T cells), where at least 10% (e.g., 20%, 30%, 40%, 50%, 60%, 70%, 80%, 90%, 95%, 97%, 99%, or substantially all) of the cell population is engineered to express a heterologous targeting construct.Alternatively, the invention features a cell population (e.g., an isolated cell population) of engineered MAIT cells (e.g., at least 10, $10^2$, $10^3$, $10^4$, $10^5$, $10^6$, $10^7$, $10^8$, $10^9$, $10^{10}$, $10^{11}$, $10^{12}$, or $10^{13}$ NK cells or NK-like T cells), where at least 10% (e.g., 20%, 30%, 40%, 50%, 60%, 70%, 80%, 90%, 95%, 97%, 99%, or substantially all) of the cell population is engineered to express a heterologous targeting construct.

**Heterologous Targeting Constructs**

Various types of $\gamma\delta$ T cells, NK cells, NK-like T cells, innate lymphoid cells, or MAIT cells can be modified to include a heterologous targeting construct to produce an engineered $\gamma\delta$ T cell, NK cell, NK-like T cell, innate lymphoid cell, or MAIT cell. The heterologous targeting construct includes an extracellular antigen-binding domain and a transmembrane domain. For example, the heterologous targeting construct may include an extracellular antigen-binding domain operatively linked to a transmembrane domain by 1-1,000 amino acid residues (e.g., by 1-10, 10-20, 20-30, 30-40, 40-50, 50-100, 100-250, 250-500, or 500-1,000 amino acid residues). In some embodiments, the antigen-binding domain is connected to a transmembrane domain by a stalk domain. In some embodiments, the extracellular antigen-binding domain, the stalk domain, and the transmembrane domain are operatively linked in an N-to-C-terminal orientation (e.g., N-antigen binding domain-stalk domain-transmembrane domain-C). In some embodiments, the extracellular antigen-binding domain, the stalk domain, and the transmembrane domain are directly linked in an N-to-C-terminal orientation.

In general, a heterologous targeting construct disclosed herein includes an antigen binding domain of a specific antibody without an intracellular signaling domain. In contrast to engineered $\alpha\beta$ T cells (e.g., CAR T cells), which are not effective without a functional intracellular domain (Ghosh et al., Nat. Med., 23: 242-251, 2017; Whilding et al. Mol. Ther., 25: 259-273, 2017; and Wilkie et al. J. Biol. Chem., 285: 25538-25544, 2010), activation of innate-like lymphocytes, such as $\gamma\delta$ T cells, can be mediated by a heterologous targeting construct without a functional intracellular domain. One of skill in the art will appreciate that the polypeptide may contain nonfunctional intracellular amino acid residues, e.g., as an extension of the transmembrane domain, which does not directly activate the engineered T cell. For example, in some aspects, the transmembrane domain may include extra residues for structural, stability, and/or expression purposes, or may have a non-functional intracellular domain. In some embodiments, no more than 50% (e.g., no more than 45%, 40%, 35%, 30%, 25%, 20%, 15%, 10%, or 5%) of the residues of the C-terminal transmembrane domain reside intracellularly.

*Antigen-binding domain*

The antigen-binding domain may be an antibody or antibody fragment engineered to specifically bind to a target. Antigen-binding domains can take the form of various structures, for example, a B cell receptor, an antibody scaffold or mimetic (e.g., an affibody, an affilin, an anticalin, an aptamer, an atrimer, a DARPin, an FN3 scaffold, a fynomer, a Kunitz domain, a pronectin, an Obody, a bicyclic peptide, a cys-knot, etc.), a single chain variable fragment (scFv), a monoclonal antibody (mAb), an antigen-binding Fragment (Fab), or T cell receptor (TCR) (FIG. 2A). The antigen-binding domain may bind to a target such as a tumor-associated antigen (TAA; e.g., a TAA expressed on a solid tumor). The TAA may be, for example, a protein or peptide antigen expressed on the surface of a tumor cell. Alternatively, TAAs include carbohydrates or gangliosides expressed on the surface of a tumor cell. In some embodiments, the TAA is an immunosuppressive antigen. In some embodiments, the antigen-binding domain is a ligand-specific receptor, as illustrated in FIG. 2B. In some embodiments, the antigen-binding domain a receptor-specific ligand, as illustrated in FIG. 2C.

In one aspect, the target binding portion of the heterologous targeting construct is a scFv. In one aspect, such antibody fragments are functional in that they retain the equivalent binding affinity, e.g., they bind the same antigen with comparable efficacy, as the IgG antibody from which it is derived. Alternatively, they can be engineered for enhanced binding affinity or weaker binding affinity as necessary, for example, to achieve optimal binding kinetics (e.g., avidity) based, for example, on the expression density of a target antigen. In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan.

In one aspect, the antigen binding domain of the heterologous targeting construct is a murine scFv antibody fragment. In another aspect, the antigen binding domain of the heterologous targeting construct is a scFv antibody fragment that is humanized compared to the murine sequence of the scFv from which it is derived. Humanization of a mouse scFv may be desired for the clinical setting, where the mouse-specific residues may induce a human-anti-mouse antigen (HAMA) response in patients who receive engineered T cell treatment.

In one aspect, the antigen binding domain portion of a heterologous targeting construct is encoded by a transgene whose sequence has been codon optimized for expression in a mammalian cell. In one aspect, entire heterologous targeting construct of the invention is encoded by a transgene having a sequence which has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, for example, the methods disclosed in U.S. Patent Nos. 5,786,464 and 6,114,148, both of which are incorporated herein by reference in their entireties.

In one aspect, the heterologous targeting construct of the invention includes a target-specific binding element antigen binding domain. The choice of moiety depends upon the type and number of ligands that define the surface of a target cell. For example, the antigen binding domain may be chosen to recognize a ligand that acts as a cell surface marker on target cells associated with a particular disease state. Examples of cell surface markers that may act as ligands for the antigen binding domain in a heterologous targeting construct of the invention include those associated with cancer, as well as viral, bacterial, parasitic infections.

In one aspect, the heterologous targeting construct-mediated T cell response can be directed to an antigen of interest by way of engineering an antigen binding domain that specifically binds a desired antigen into the heterologous targeting construct. The antigen binding domain can be any domain that binds to the antigen including, but not limited to, a monoclonal antibody, a polyclonal antibody, a recombinant antibody, a human antibody, a humanized antibody, and a functional fragment thereof, including but not limited to a single-domain antibody such as a heavy chain variable domain (VH), a light chain variable domain (VL) and a variable domain of camelid derived nanobody, and to an alternative scaffold known in the art to function as antigen binding domain, such as a recombinant fibronectin domain, and the like. In some instances, it is beneficial for the antigen binding domain to be derived from the same species in which the heterologous targeting construct will ultimately be used in. For example, for use in humans, it may be beneficial for the antigen binding domain of the heterologous targeting construct to include human or humanized residues for the antigen binding domain of an antibody or antibody fragment.

In some embodiments of any aspect of the invention, a heterologous targeting construct includes features an antigen-binding domain that is a humanized antibody or antigen-binding fragment thereof. A humanized antibody can be produced using a variety of techniques known in the art, including but not limited to, CDR-grafting (see, e.g., European Patent No. EP 239,400; PCT Publication No. WO 91/09967; and U.S. Patent Nos. 5,225,539, 5,530,101, and 5,585,089, each of which is incorporated herein in its entirety by reference), veneering or resurfacing (see, e.g., European Patent Nos. EP 592,106 and EP 519,596, each of which is incorporated herein by its entirety by reference), chain shuffling (see, e.g., U.S. Patent No. 5,565,332, which is incorporated herein in its entirety by reference), and techniques disclosed in, e.g., U.S. Patent Application Publication No. 2005/0042664, U.S. Patent Application Publication No. 2005/0048617, U.S. Patent Nos. 6,407,213 and 5,766,886, and International Publication No. WO 93/17105, each of which is incorporated herein in its entirety by reference. Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. See, e.g., U.S. Patent No. 5,585,089, which are incorporated herein by reference in its entirety.

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments include one or more CDRs from nonhuman immunoglobulin molecules and framework regions wherein the amino acid residues including the framework are derived completely or mostly from human germline. Multiple techniques for humanization of antibodies or antibody fragments are well-known in the art and can essentially be performed following the methods of Jones et al., Nature, 1986, 321:522-525; Riechmann et al., Nature, 1988, 332:323-327; and Verhoeyen et al., Science, 1988, 239:1534-1536, each of which is incorporated herein by reference in its entirety, by substituting rodent CDRs or CDR sequences for the corresponding sequences of a human antibody, i.e., CDR-grafting. In such humanized antibodies and antigen-binding fragments thereof, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies.

In some aspects, the portion of a heterologous targeting construct of the invention that includes an antibody fragment is humanized with retention of high affinity for the target antigen and other favorable biological properties. According to one aspect of the invention, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

In some instances, scFvs can be prepared according to method known in the art (see, for example, Bird et al., Science, 1988, 242:423-426 and Huston et al., Proc. Natl. Acad. Sci. USA, 1988, 85:5879-5883; each of which is incorporated herein by reference in its entirety). ScFv molecules can be produced by linking VH and VL regions together using flexible polypeptide linkers. The scFv molecules include a linker (e.g., a Ser-Gly linker) with an optimized length and/or amino acid composition. The linker length can greatly affect how the variable regions of an scFv fold and interact. In fact, if a short polypeptide linker is employed (e.g., between 5-10 amino acids), intrachain folding is prevented. Interchain folding is also required to bring the two variable regions together to form a functional epitope binding site. For examples of linker orientation and size see, e.g., Hollinger et al. Proc Natl Acad. Sci. USA, 1993, 90:6444-6448, U.S. Publication Nos. 2005/0100543, 2005/0175606, 2007/0014794, and International Patent Publication Nos. WO 2006/020258 and WO 2007/024715, which are incorporated herein by reference.

An scFv can include a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may include any naturally occurring amino acid. In some embodiments, the linker sequence includes amino acids glycine and serine. In another embodiment, the linker sequence includes sets of glycine and serine repeats such as (GGGGS)n, where n is a positive integer equal to or greater than 1 (e.g., 1, 2, 3, 4, 5 or more). Variation in the linker length may retain or enhance activity, giving rise to superior efficacy in binding and activity.

Kinetics of cytolysis of a target cell by an engineered $\gamma\delta$ T cell of the invention is determined, in part, by the binding affinity of the antigen-binding domain. Any of the antigen-binding domains provided herein can be modified according to known methods to enhance or reduce binding affinity to a particular target, as desired. In some embodiments, the antigen-binding domain has a binding affinity or dissociation constant ($K_D$) for its antigen from $10^{-4}$ M to $10^{-10}$ M (e.g., from $10^{-4}$ M to $10^{-5}$ M, from $10^{-5}$ M to $10^{-6}$ M, from $10^{-6}$ M to $10^{-7}$ M, from $10^{-7}$ M to $10^{-8}$ M, from $10^{-8}$ M to $10^{-9}$ M, from $10^{-9}$ M to $10^{-10}$ M, e.g., from $10^{-5}$M to $10^{-9}$ M, from $10^{-5}$ M to $10^{-8}$ M, from $10^{-5}$M to $10^{-7}$ M, from $10^{-5}$ M to $10^{-6}$ M, from $10^{-6}$ M to $10^{-10}$ M, from $10^{-6}$ M to $10^{-9}$ M, from $10^{-6}$ M to $10^{-8}$ M, from $10^{-6}$ M to $10^{-7}$ M, from $10^{-7}$ M to $10^{-10}$ M, from $10^{-7}$ M to $10^{-9}$ M, from $10^{-7}$ M to $10^{-8}$ M, from $10^{-8}$ M to $10^{-10}$ M, from $10^{-8}$ M to $10^{-9}$ M, or from $10^{-9}$ M to $10^{-10}$ M, as measured under standard physiological temperatures and pressures, e.g., by surface plasmon resonance, e.g., BIAcore).

In addition to binding affinity of the antigen-binding domain of the engineered $\gamma\delta$ T cell to a target cell, avidity interactions also play a role in effective binding and subsequent lysis of target cells. Avidity is dictated by (a) the binding affinity of the antigen-binding domain and (b) the number of interactions between antigen-binding domain and antigen along a given interface between T cell and target. In some embodiments, an engineered $\gamma\delta$ T cell contains, on its surface, from $10^2$ to $10^6$ antigen-binding domains

(e.g., from $10^2$ to $10^3$, from $10^3$ to $10^4$, from $10^4$ to $10^5$, from $10^5$ to $10^6$, from $10^2$ to $10^4$, from $10^2$ to $10^5$, from $10^3$ to $10^4$, from $10^3$ to $10^5$, from $10^3$ to $10^6$, from $10^4$ to $10^5$, from $10^4$ to $10^6$, or from $10^5$ to $10^6$) antigen-binding domains.

*Stalk Domain*

The heterologous targeting constructs of the present invention can include a stalk domain located between the transmembrane domain and the extracellular antigen-binding domain. In some embodiments, the stalk domain includes one or more of the domains selected from the group consisting of a CD8 stalk, an IgG hinge-heavy constant (CH) domain (e.g., an IgG1 hinge-CH$_2$ domain, an IgG1 hinge-CH$_3$ domain, or an IgG1 hinge-CH$_2$-CH$_3$ domain), a (G$_4$S)$_3$ hinge, an IgG1 hinge, a CD7 stalk, an IgD hinge-CH$_2$ domain, an IgD hinge-CH$_3$ domain, an IgD hinge-CH$_2$-CH$_3$ domain, an IgG4 hinge-CH$_2$ domain, an IgG4 hinge-CH$_3$ domain, an IgG4 hinge-CH$_2$-CH$_3$ domain, or an Fc$\epsilon$RI stalk. The stalk may provide flexibility between the extracellular and transmembrane domains and may assist in target recognition. It would be understood by one of skill in the art that the stalk domain may include one or more additional amino acids adjacent to the extracellular or transmembrane region, e.g., one or more amino acid associated with the extracellular or transmembrane regions of the protein from which the stalk was derived (e.g., 1, 2, 3, 4, 5, 6, 7, 8, 9, 10, 11, 12, 13, 14, or 15 amino acids of the extracellular or transmembrane regions). The stalk may optionally include one or more linkers such as (GGGGS)$_n$ or GGGGSGGGGS (SEQ ID NO: 1).

*Transmembrane Domain*

In various embodiments of any aspect of the invention, a heterologous targeting construct can be designed to include a transmembrane domain that is attached to the extracellular domain(s). A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10, 11, 12, 13, 14, or 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the heterologous targeting construct. In some instances, the transmembrane domain can be selected or modified by amino acid substitution to avoid binding of such domains to the transmembrane domains of the same or different surface membrane proteins, e.g., to minimize interactions with other members of the receptor complex. Thus, in some instances, the transmembrane domain does not substantially propagate signal 1 activation and/or signal 2 activation (co-stimulation).

Alternatively, a transmembrane domain can be selected for its ability to bind to, induce clustering of, activate, phosphorylate, dephosphorylate, or otherwise interact with other proteins (e.g., endogenous proteins, e.g., endogenous membrane-associated proteins, such as transmembrane proteins). For instance, in some embodiments, the transmembrane domain may associate with a co-stimulatory protein, thereby indirectly activating the cell (e.g., the $\gamma\delta$ T cell) by propagating a signal 2 co-stimulatory signal. In particular embodiments, a transmembrane domain is derived from a transmembrane portion of NKG2D, which may associate with endogenously expressed DAP10 to propagate signal 2 activation (co-stimulation) of the host cell. In such instances, the heterologous targeting construct does not have a functional intracellular domain capable of activating the cell. For example, the heterologous targeting construct may be devoid of an intracellular domain, or it may contain an inert intracellular domain, which does not transmit signal 1 or signal 2 activation. For example, a transmembrane domain that can propagate signal 2 by recruiting or associating with an endogenous co-stimulatory molecule may be a terminal transmembrane domain (e.g., there are no additional domains attached to one of its termini).

In one aspect, the transmembrane domain is capable of hetero- or homo-dimerization with another heterologous targeting construct on the $\gamma\delta$ T cell surface. In a different aspect, the amino acid sequence of the transmembrane domain may be modified or substituted so as to minimize interactions with the binding domains of the native binding partner present in the same engineered T cell.

The transmembrane domain may be derived either from a natural or from a recombinant source. Where the source is natural, the domain may be derived from any membrane-bound or transmembrane protein. A transmembrane domain of particular use in this invention may include at least the transmembrane region(s) of e.g., the alpha, beta or zeta chain of the T cell receptor, CD28, CD3 epsilon, CD45, CD4, CD5, CD8, CD9, CD11a, CD11b, CD11c, CD11d, CD16, CD18, CD22, CD29, CD33, CD37, CD64, CD80, CD86, CD94, CD134, CD137, CD154, CD7, CD3 zeta, CD71, Fc gamma receptor (FcyR), or NKG2D. In some embodiments, the transmembrane domain may include, for example, a CD8 transmembrane domain, a CD4 transmembrane domain, a CD3$\zeta$ transmembrane domain, or a CD28 transmembrane domain. In some embodiments, the transmembrane domain is a terminal transmembrane domain (e.g., there are no additional domains attached to one of its termini).

In some instances, the transmembrane domain can be attached to the extracellular region of the heterologous targeting construct, e.g., the antigen binding domain of the heterologous targeting construct, via a hinge, e.g., a hinge from a human protein. For example, in one embodiment, the hinge can be a human Ig (immunoglobulin) hinge, e.g., an IgG1 hinge, an IgG4 hinge, an IgD hinge, or a CD8$\alpha$ hinge.

In one embodiment, the transmembrane domain is recombinant, in which case it will include predominantly hydrophobic residues such as leucine and valine. In one aspect, a triplet of phenylalanine, tryptophan and valine can be found at each end of a recombinant transmembrane domain.

Optionally, a short polypeptide linker, e.g., between two and ten amino acids in length, may form the linkage between the transmembrane domain and the cytoplasmic region of the heterologous targeting construct. A glycine-serine doublet is an example of a suitable linker. For example, in one aspect, the linker includes the amino acid sequence of (GGGGS)$_n$ or GGGGSGGGGS (SEQ ID NO: 1).

**Methods of Harvesting and Expanding $\gamma\delta$ T Cells**

Engineered $\gamma\delta$ T cells of the invention can be derived from any suitable autologous or allogeneic $\gamma\delta$ T cell or population thereof. In some embodiments, suitable $\gamma\delta$ T cells for use as a source for the presently described engineered $\gamma\delta$ T cells include V$\delta$1 cells, V$\delta$2 cells, V$\delta$3 cells, V$\delta$5 cells, and V$\delta$8 cells. In some embodiments, the population of engineered $\gamma\delta$ T cell is derived from a population of V$\delta$1 cells, V$\delta$3 cells, V$\delta$5 cells, or V$\delta$8 cells.

For example, provided herein are methods for separating and expanding V$\delta$1 cells from a non-haematopoietic tissue, such as skin or gut. For example, V$\delta$1 cells can be isolated from human skin biopsies as described in U.S. 2018/0312808, which is hereby incorporated by reference in its entirety and specifically for methods of isolating V$\delta$1 cells from tissue.

In other embodiments, suitable $\gamma\delta$ T cells can be derived from blood (e.g., peripheral blood). Methods of isolating and expanding V$\delta$1 cells from blood include those described, for example, in U.S.

Patent No. 9,499,788 and International Patent Publication No. WO 2016/198480, each of which is incorporated herein by reference in its entirety. In some embodiments, suitable $\gamma\delta$ T cells can be derived from tumor tissue (e.g., tumor-infiltrating $\gamma\delta$ T cells).Alternatively, suitable $\gamma\delta$ T cells that can be engineered to express a heterologous targeting construct can be derived from non-haematopoietic tissue according to methods described below.

*Isolation and expansion of y$\delta$ T cells from blood*

In some embodiments, the engineered $\gamma\delta$ T cells of the present invention are derived from blood (e.g., peripheral blood) of a subject. For example, engineered $\gamma\delta$ T cells may be derived from blood-derived V$\delta$2 cells or blood-derived V$\delta$1 cells.

In some embodiments, peripheral blood mononuclear cells (PBMCs) can be obtained from a subject according to any suitable method known in the art. PBMCs can be cultured in the presence of aminobisphosphonates (e.g., zoledronic acid), synthetic phosphoantigens (e.g., bromohydrin pyrophosphate; BrHPP), 2M3B1 PP, or 2-methyl-3-butenyl-1-pyrophosphate in the presence of IL-2 for one-to-two weeks to generate an enriched population of V$\delta$2 cells. Alternatively, immobilized anti-TCR$\gamma\delta$ (e.g., pan TCR$\gamma\delta$) can induce preferential expansion of V$\delta$2 cells from a population of PBMCs in the presence of IL-2, e.g., for approximately 14 days. In some embodiments, preferential expansion of V$\delta$2 cells from PBMCs can be achieved upon culture of immobilized anti-CD3 antibodies (e.g., OKT3) in the presence of IL-2 and IL-4. In some embodiments, the aforementioned culture is maintained for about seven days prior to subculture in soluble anti-CD3, IL-2, and IL-4. Alternatively, artificial antigen presenting cells can be used to promote preferential expansion of $\gamma\delta$ T cells, such as V$\delta$2 cells. For example, PBMC-derived $\gamma\delta$ T cells cultured in the presence of irradiated aAPC, IL-2, and/or IL-21 can expand to generate a population of $\gamma\delta$ T cells including a high proportion of V$\delta$2 cells, moderate proportion of V$\delta$1 cells, and some double negative cells. In some embodiments of the aforementioned methods, PBMCs can be pre-enriched or post-enriched (e.g. through positive selection with TCR$\gamma\delta$-specific agents or negative selection of TCR$\alpha\beta$-specific agents). Such methods and other suitable methods for expansion of $\gamma\delta$ T cells, such as V$\delta$2 cells, are described in detail by Deniger et al., Frontiers in Immunology 2014, 5, 636: 1-10, which is incorporated herein by reference in its entirety.

In some embodiments, V$\delta$1 T cells can be engineered to express a heterologous targeting construct. Any suitable method of obtaining a population of V$\delta$1 T cells can be used. For example, Almeida et al. (Clinical Cancer Research 2016, 22, 23; 5795-5805), incorporated herein by reference in its entirety, provides suitable methods of obtaining a population of V$\delta$1 T cells that can be engineered to express a heterologous targeting construct described herein. For example, in some embodiments, PBMCs are pre-enriched using magnetic bead sorting, which can yield greater than 90% $\gamma\delta$ T cells. These cells can be cultured in the presence of one or more factors (e.g., TCR agonists, co-receptor agonists, and/or cytokines, e.g., IL-4, IL-15, and/or IFN-$\gamma$) in gas-permeable bioreactor bags for up to 21 days or more. Variations of this method, and other methods of obtaining V$\delta$1 T cells are suitable as part of the present invention. For example, blood-derived V$\delta$1 T cells can alternatively be obtained using methods described, for example, in U.S. Patent No. 9,499,788 and International Patent Publication No. WO 2016/198480, each of which is incorporated herein by reference in its entirety, as well as WO2017197347, and WO2016081518 (US Publ. No. 20160175338), each of which is incorporated herein by reference in its entirety.

*Separation and Expansion of non-haematopoietic tissue-resident $\gamma\delta$ T cells from non-haematopoietic tissue*

Non-haematopoietic tissue-resident $\gamma\delta$ T cells obtained as described below are likely to be suitable vehicles for heterologous targeting constructs described herein, as they can exhibit good tumor penetration and retention capabilities. More detailed methods for isolation and expansion of non-haematopoietic tissue-resident $\gamma\delta$ T cells can be found, for example, in GB Application No. 1707048.3 (WO2018/202808) and in International Patent Publication No. WO 2017/072367 (US Publ. No. 20180312808), each of which is incorporated herein by reference in its entirety.

Non-haematopoietic tissue-resident γδ T cells (e.g., skin-derived γδ T cells and/or non-Vδ2 T cells, e.g., Vδ1 T cells and/or DN T cells) can be isolated from any human or non-human animal non-haematopoietic tissue that can be removed from a patient to obtain cells suitable for engineering according to the methods of the present invention. In some embodiments, the non-haematopoietic tissue from which the γδ T cells are derived and expanded is skin (e.g., human skin), which can be obtained by methods known in the art. In some embodiments, the skin is obtained by punch biopsy. Alternatively, the methods of isolation and expansion of γδ T cells provided herein can be applied to the gastrointestinal tract (e.g., colon), mammary gland, lung, prostate, liver, spleen, and pancreas. The γδ T cells may also be resident in human cancer tissues, e.g., tumors of the breast or prostate. In some embodiments, the γδ T cells may be from human cancer tissues (e.g., solid tumor tissues). In other embodiments, the γδ T cells may be from non-haematopoietic tissue other than human cancer tissue (e.g., a tissue without a substantial number of tumor cells). For example, the γδ T cells may be from a region of skin (e.g., healthy skin) separate from a nearby or adjacent cancer tissue.

The γδ T cells that are dominant in the blood are primarily Vδ2 T cells, while the γδ T cells that are dominant in the non-haematopoietic tissues are primarily Vδ1 T cells, such that Vδ1 T cells include about 70-80% of the non-haematopoietic tissue-resident γδ T cell population. However, some Vδ2 T cells are also found in non-haematopoietic tissues, e.g. in the gut, where they can include about 10-20% of γδ T cells. Some γδ T cells that are resident in non-haematopoietic tissues express neither Vδ1 nor Vδ2 TCR and we have named them double negative (DN) γδ T cells. These DN γδ T cells are likely to be mostly Vδ3-expressing with a minority of Vδ5-expressing T cells. Therefore, the γδ T cells that are ordinarily resident in non-haematopoietic tissues and that are expanded by the method of the invention are preferably non-Vδ2 T cells, e.g. Vδ1 T cells, with the inclusion of a smaller amount of DN γδ T cells.

In some embodiments, a critical step is the deliberate separation, e.g., after some days or weeks of culture, of non-haematopoietic tissue-resident T cells (e.g., within a mixed lymphocyte population, which may for example include αβ cells, natural killer (NK) cells, B cells, and γ2 and non-γ2 T cells) away from the non-haematopoietic cells (e.g. stromal cells, particularly fibroblasts) of the tissue from which the T cells were obtained. This permits the preferential and rapid expansion over the following days and weeks of non-haematopoietic tissue-derived Vδ1 T cells and DN γδ T cells.

In general, non-haematopoietic tissue-resident γδ T cells are capable of spontaneously expanding upon removal of physical contact with stromal cells (e.g., skin fibroblasts). Thus, the scaffold-based culture methods described above can be used to induce such separation, resulting in de-repression of the γδ T cells to trigger expansion. Accordingly, in some embodiments, no substantial TCR pathway activation is present during the expansion step (e.g., no exogenous TCR pathway activators are included in the culture). Further, the invention provides methods of expanding non-haematopoietic tissue-resident γδ T cells, wherein the methods do not involve contact with feeder cells, tumor cells, and/or antigen-presenting cells.

Expansion protocols involve culturing non-haematopoietic tissue-resident γδ T cells in the presence of effective cocktails of biological factors to support efficient γδ T cell expansion. In one embodiment, the method of expanding γδ T cells includes providing a population of γδ T cells obtained from a non-haematopoietic tissue (e.g., a separated population of non-haematopoietic tissue-derived γδ T cells, e.g., a population separated according to the methods described herein) and culturing the γδ T cells in the presence of IL-2, IL-4, IL-15, and/or IL-21. These cytokines or analogues thereof can be cultured with the cells for a duration (e.g., at least 5 days, at least 6 days, at least 7 days, at least 8 days, at least 9 days, at least 10 days, at least 11 days, at least 12 days, at least 13 days, at least 14 days, at least 21 days, at least 28 days, or longer, e.g., from 5 days to 40 days, from 7 days to 35 days, from 14 days 28 days, or about 21 days) in an amount effective to produce an expanded population of γδ T cells.

*Expanded γδ T cell populations*

The expanded population of γδ T cells is greater in number than the separated population of γδ T cells prior to the expansion step (e.g., at least 2-fold in number, at least 3-fold in number, at least 4-fold in number, at least 5-fold in number, at least 6-fold in number, at least 7-fold in number, at least 8-fold in number, at least 9-fold in number, at least 10-fold in number, at least 15-fold in number, at least 20-fold in number, at least 25-fold in number, at least 30-fold in number, at least 35-fold in number, at least 40-fold in number, at least 50-fold in number, at least 60-fold in number, at least 70-fold in number, at least 80-fold in number, at least 90-fold in number, at least 100-fold in number, at least 200-fold in number, at least 300-fold in number, at least 400-fold in number, at least 500-fold in number, at least 600-fold in number, at least 700-fold in number, at least 800-fold in number, at least 900-fold in number, at least 1,000-fold in number at least 5,000-fold in number, at least 10,000-fold in number, or more relative to the separated population of γδ T cells prior to the expansion step). Thus, large populations of γδT cells (e.g., skin-derived γδ T cells and/or non-Vδ2 T cells, e.g., Vδ1 T cells and/or DN T cells) can be expanded at high rates. In some embodiments, the expansion step described herein expands the γδ T cells at a low population doubling time, which is given by the following equation:

$$DoublingTime = \frac{duration * \log(2)}{\log(FinalConcentration) - \log(InitialConcentration)}$$

Given the information provided herein, a skilled artisan will recognize that the invention provides methods of expanding $\gamma\delta$ T cells (e.g., engineered $\gamma\delta$ T cells or $\gamma\delta$ T cells that are expanded and/or selected for engineering to express a heterologous targeting construct) at a population doubling time of less than 5 days (e.g., less than 4.5 days, less than 4.0 days, less than 3.9 days, less than 3.8 days, less than 3.7 days, less than 3.6 days, less than 3.5 days, less than 3.4 days, less than 3.3 days, less than 3.2 days, less than 3.1 days, less than 3.0 days, less than 2.9 days, less than 2.8 days, less than 2.7 days, less than 2.6 days, less than 2.5 days, less than 2.4 days, less than 2.3 days, less than 2.2 days, less than 2.1 days, less than 2.0 days, less than 46 hours, less than 42 hours, less than 38 hours, less than 35 hours, less than 32 hours).

In some embodiments, $\gamma\delta$ T cells (e.g., engineered $\gamma\delta$ T cells or $\gamma\delta$ T cells that are expanded and/or selected for engineering to express a heterologous targeting construct) isolated and expanded by the methods provided herein can have a phenotype well-suited for anti-tumor efficacy. In some embodiments, the expanded population of yb T cells has a greater mean expression of CD27 than a reference population (e.g., the separated population of $\gamma\delta$ T cells prior to the expansion step). In some embodiments, the expanded population of yb T cells has a mean expression of CD27 that is at least 2-fold relative to the separated population of $\gamma\delta$ T cells (e.g., at least 3-fold, at least 4-fold, at least 5-fold, at least 6-fold, at least 7-fold, at least 8-fold, at least 9-fold, at least 10-fold, at least 15-fold, at least 20-fold, at least 25-fold, at least 30-fold, at least 40-fold, at least 50-fold, at least 60-fold, at least 70-fold, at least 80-fold, at least 90-fold, at least 100-fold, at least 150-fold, at least 200-fold, at least 300-fold, at least 400-fold, at least 500-fold, at least 600-fold, at least 700-fold, at least 800-fold, at least 900-fold, at least 1,000-fold, at least 5,000-fold, at least 10,000-fold, at least 20,000-fold, or more, relative to the separated population of $\gamma\delta$ T cells).

A distinct portion of the expanded population of $\gamma\delta$ T cells (e.g., engineered $\gamma\delta$ T cells or $\gamma\delta$ T cells that are expanded and/or selected for engineering to express a heterologous targeting construct) may upregulate CD27, while another portion is CD27^low or CD27^-. In this case, the frequency of CD27^+ cells in the expanded population relative to the separated population of $\gamma\delta$ T cells may be greater. For example, the expanded population of $\gamma\delta$ T cells may have at least a 5% greater frequency of CD27^+ cells relative to that of the separated population of $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% greater frequency of CD27^+ cells relative to that of the separated population of $\gamma\delta$ T cells prior to expansion). In some embodiments, the number of CD27^+ cells in the expanded population relative to the separated population of $\gamma\delta$ T cells may be increased. For example, the expanded population of $\gamma\delta$ T cells may have at least 2-fold the number of CD27^+ cells relative to the separated population of $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% greater frequency of CD27^+ cells relative to that of the separated population of $\gamma\delta$ T cells prior to expansion).

Methods of expansion as provided herein, in some embodiments, yield an expanded population of $\gamma\delta$ T cells (e.g., engineered $\gamma\delta$ T cells or $\gamma\delta$ T cells that are expanded and/or selected for engineering to express a heterologous targeting construct) having a low expression of TIGIT, relative to a reference population (e.g., the separated population of $\gamma\delta$ T cells prior to the expansion step). In some embodiments, the expanded population of $\gamma\delta$ T cells has a lower mean expression of TIGIT than a reference population (e.g., the separated population of $\gamma\delta$ T cells prior to the expansion step). In some embodiments, the expanded population of $\gamma\delta$ T cells has a mean expression of TIGIT that is at least 10% less than the separated population of $\gamma\delta$ T cells (e.g., at least 20% less, at least 30% less, at least 40% less, at least 50% less, at least 60% less, at least 70% less, at least 80% less, at least 90% less, or up to 100% less than the separated population of $\gamma\delta$ T cells).

A distinct portion of the expanded population of $\gamma\delta$ T cells (e.g., engineered $\gamma\delta$ T cells or $\gamma\delta$ T cells that are expanded and/or selected for engineering to express a heterologous targeting construct) may express TIGIT, e.g., high levels of TIGIT, while another portion is TIGIT^low or TIGIT^-. In this case, the frequency of TIGIT^+ cells in the expanded population relative to the separated population of $\gamma\delta$ T cells may be lower. For example, the expanded population of $\gamma\delta$ T cells may have at least a 5% lower frequency of TIGIT^+ cells relative to that of the separated population of $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% lower frequency of TIGIT^+ cells relative to that of the separated population of $\gamma\delta$ T cells prior to expansion). In some embodiments, the number of TIGIT^+ cells in the expanded population relative to the separated population of $\gamma\delta$ T cells prior to expansion may be lower. For example, the expanded population of $\gamma\delta$ T cells may have at least 10% fewer TIGIT^+ cells relative to the number of TIGIT^+ cells in the separated population of $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% fewer TIGIT$^+$ cells relative to the number of TIGIT$^+$ cells in the separated population of $\gamma\delta$ T cells prior to expansion).

In some embodiments, the expanded population of $\gamma\delta$ T cells (e.g., engineered $\gamma\delta$ T cells or $\gamma\delta$ T cells that are expanded and/or selected for engineering to express a heterologous targeting construct) has a high number or frequency of CD27$^+$ cells and a low frequency of TIGIT$^+$ cells. In some embodiments, the expanded population of $\gamma\delta$ T cells has a high frequency of CD27$^+$TIGIT cells relative to a reference population (e.g., relative to a separated population of $\gamma\delta$ T cells prior to expansion). For instance, the expanded population of $\gamma\delta$ T cells may have at least a 5% greater frequency of CD27$^+$ TIGIT cells relative to that of the separated population of $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% greater frequency of CD27$^+$ TIGIT cells relative to that of the separated population of $\gamma\delta$ T cells prior to expansion). In some embodiments, the number of CD27$^+$ TIGIT cells in the expanded population relative to the separated population of $\gamma\delta$ T cells may be increased. For example, the expanded population of $\gamma\delta$ T cells may have at least 2-fold the number of CD27$^+$ TIGIT cells relative to the separated population of $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% greater frequency of CD27$^+$TIGIT$^-$cells relative to that of the separated population of $\gamma\delta$ T cells prior to expansion).

In some instances, the mean expression of TIGIT on a population of CD27$^+$ $\gamma\delta$ T cells in an expanded population of $\gamma\delta$ T cells (e.g., engineered $\gamma\delta$ T cells or $\gamma\delta$ T cells that are expanded and/or selected for engineering to express a heterologous targeting construct) is low relative to a reference population. In some embodiments, the expanded population of CD27$^+$ $\gamma\delta$ T cells has a lower mean expression of TIGIT than a reference population (e.g., the separated population of CD27$^+$ $\gamma\delta$ T cells prior to the expansion step). In some embodiments, the expanded population of CD27$^+$ $\gamma\delta$ T cells has a mean expression of TIGIT that is at least 10% less than the separated population of CD27$^+$ $\gamma\delta$ T cells (e.g., at least 20% less, at least 30% less, at least 40% less, at least 50% less, at least 60% less, at least 70% less, at least 80% less, at least 90% less, or up to 100% less than the separated population of CD27$^+$ $\gamma\delta$ T cells).

Additionally or alternatively, the median expression of CD27 on a population of TIGIT $\gamma\delta$ T cells in an expanded population of $\gamma\delta$ T cells (e.g., engineered $\gamma\delta$ T cells or $\gamma\delta$ T cells that are expanded and/or selected for engineering to express a heterologous targeting construct) is high relative to a reference population. For example, the expanded population of TIGIT$^-$ $\gamma\delta$ T cells may have at least a 5% greater frequency of CD27$^+$ cells relative to that of the separated population of TIGIT$^-$ $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% greater frequency of CD27$^+$ cells relative to that of the separated population of TIGIT $\gamma\delta$ T cells prior to expansion). In some embodiments, the number of CD27$^+$ cells in the expanded population relative to the separated population of TIGIT$^-$ $\gamma\delta$ T cells may be increased. For example, the expanded population of TIGIT$^-$ $\gamma\delta$ T cells may have at least 2-fold the number of CD27$^+$ cells relative to the separated population of TIGIT $\gamma\delta$ T cells prior to expansion (e.g., at least a 10%, at least a 15%, at least a 20%, at least a 25%, at least a 30%, at least a 35%, at least a 40%, at least a 45%, at least a 50%, at least a 60%, at least a 70%, at least an 80%, at least a 90%, or up to 100% greater frequency of CD27$^+$ cells relative to that of the separated population of TIGIT$^-$ $\gamma\delta$ T cells prior to expansion).

An increase or decrease in expression of other markers can be additionally or alternatively used to characterize one or more expanded populations of $\gamma\delta$ T cells (e.g., engineered $\gamma\delta$ T cells or $\gamma\delta$ T cells that are expanded and/or selected for engineering to express a heterologous targeting construct), including CD124, CD215, CD360, CTLA4, CD1b, BTLA, CD39, CD45RA, Fas Ligand, CD25, ICAM-1, CD31, KLRG1, CD30, CD2, NKp44, NKp46, ICAM-2, CD70, CD28, CD103, NKp30, LAG3, CCR4, CD69, PD-1, and CD64. In some instances, the expanded population of $\gamma\delta$ T cells has a greater, equal or lower mean expression of one or more of the markers selected from the group consisting of CD124, CD215, CD360, CTLA4, CD1b, BTLA, CD39, CD45RA, Fas Ligand, CD25, ICAM-1, CD31, KLRG1, CD30, and CD2, relative to the separated population of $\gamma\delta$ T cells, e.g., prior to expansion. Additionally or alternatively, the expanded population of $\gamma\delta$ T cells may have a greater, equal or lower frequency of cells expressing one or more of the markers selected from the group consisting of CD124, CD215, CD360, CTLA4, CD1b, BTLA, CD39, CD45RA, Fas Ligand, CD25, ICAM-1, CD31, KLRG1, CD30, and CD2, relative to the separated population of $\gamma\delta$ T cells. In some embodiments, the expanded population of $\gamma\delta$ T cells has a greater, equal or lower mean expression of one or more of the markers selected from the group consisting of NKp44, NKp46, ICAM-2, CD70, CD28, CD103, NKp30, LAG3, CCR4, CD69, PD-1, and CD64, relative to the separated population of $\gamma\delta$ T cells. The expanded population may similarly have a greater, equal or lower frequency of cells expressing one or more of the markers selected from the group consisting of NKp44, NKp46, ICAM-2, CD70, CD28, CD103, NKp30, LAG3, CCR4, CD69, PD-1, and CD64, relative separated reference population of $\gamma\delta$ T cells.

Numerous basal culture media suitable for use in the proliferation of $\gamma\delta$ T cells are available, in particular complete media, such as AIM-V, Iscoves medium and RPMI-1640 (Life Technologies). The medium may be supplemented with other media factors, such as serum, serum proteins and selective agents, such as antibiotics. For example, in some embodiments, RPMI-1640 medium containing 2 mM glutamine, 10% FBS, 10 mM HEPES, pH 7.2, 1% penicillin-streptomycin, sodium pyruvate (1 mM; Life Technologies), non-essential amino acids (e.g. 100 $\mu$M Gly, Ala, Asn, Asp, Glu, Pro and Ser; 1X MEM non-essential amino acids Life Technologies), and 10 $\mu$l/L $\beta$-mercaptoethanol. Conveniently, cells are cultured at 37°C in a humidified atmosphere containing 5% $CO_2$ in a suitable culture medium.

The $\gamma\delta$ T cells may be cultured as described herein in any suitable system, including stirred tank fermenters, airlift fermenters, roller bottles, culture bags or dishes, and other bioreactors, such as hollow fiber bioreactors. The use of such systems is well-known in the art. General methods and techniques for culture of lymphocytes are well-known in the art.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected.

## V. Pharmaceutical Compositions and Methods of Treatment

The engineered lymphocytes (e.g., $\gamma\delta$ T cells, NK cells, NK-like T cells, innate lymphoid cells, or MAIT cells) described herein (e.g., engineered cells (e.g., $\gamma\delta$ T cells) having a heterologous targeting construct) may be used as a medicament, for example, as an adoptive T cell therapy. Such use involves the transfer of lymphocytes (e.g., $\gamma\delta$ T cells) obtained by the method of the invention into a patient. The therapy may be autologous, i.e., the lymphocytes (e.g., $\gamma\delta$ T cells) may be transferred back into the same patient from which they were obtained, or the therapy may be allogeneic, i.e., the lymphocytes (e.g., $\gamma\delta$ T cells) from one person may be transferred into a different patient. In instances involving allogeneic transfer, the lymphocytes (e.g., $\gamma\delta$ T cells) may be substantially free of $\alpha\beta$ T cells. For example, $\alpha\beta$ T cells may be depleted from the lymphocyte (e.g., $\gamma\delta$ T cell) population, e.g., after expansion, using any suitable means known in the art (e.g., by negative selection, e.g., using magnetic beads).

In some embodiments in which $\gamma\delta$ T cells are engineered to express a heterologous targeting construct, the $\gamma\delta$ T cells are V$\delta$1 cells, V$\delta$2 cells, V$\delta$3 cells, V$\delta$5 cells, or V$\delta$8 cells). A method of treatment may include; providing a sample of endogenous $\gamma\delta$ T cells from a patient; culturing the $\gamma\delta$ T cells from the sample in the presence of a vector carrying a polynucleotide encoding a heterologous targeting construct to generate a population of engineered $\gamma\delta$ T cells expressing the heterologous targeting construct (e.g., an expanded population of engineered $\gamma\delta$ T cells expressing the heterologous targeting construct); and administering the population of $\gamma\delta$ T cells to a recipient patient. In some embodiments, the polynucleotide encoding a heterologous targeting construct is delivered to the endogenous $\gamma\delta$ T cells through electroporation or any other suitable method of transfection known in the art or described herein.

The patient or subject to be treated may be a human cancer patient (e.g., a human cancer patient being treated for a solid tumor) or a virus-infected patient (e.g., a CMV-infected or HIV infected patient). In some instances, the patient has and/or is being treated for a solid tumor.

As $\gamma\delta$ T cells are non-MHC restricted, they do not recognize a host into which they are transferred as foreign, which means that they are less likely to cause graft-versus-host disease. This means that they can be used "off the shelf" and transferred into any recipient, e.g., for allogeneic adoptive T cell therapy.

In some embodiments, $\gamma\delta$ T cells of the invention express NKG2D and respond to a NKG2D ligand (e.g. MICA), which is strongly associated with malignancy. They also express a cytotoxic profile in the absence of any activation and are therefore likely to be effective at killing tumor cells. For example, the engineered $\gamma\delta$ T cells obtained as described herein may express one or more, preferably all of IFN-y, TNF-$\alpha$, GM-CSF, CCL4, IL-13, granulysin, granzyme A and B, and perforin in the absence of any activation. IL-17A may not be expressed.

Pharmaceutical compositions may include engineered lymphocytes (e.g., $\gamma\delta$ T cells) as described herein in combination with one or more pharmaceutically or physiologically acceptable carrier, diluents, or excipients. Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Cryopreservation solutions which may be used in the pharmaceutical compositions of the invention include, for example, DMSO. Compositions can be formulated, e.g., for intravenous administration.

In one embodiment, the pharmaceutical composition is substantially free of, e.g., there are no detectable levels of a contaminant, e.g., of endotoxin or mycoplasma.

In some instances, a therapeutically effective amount of engineered lymphocytes (e.g., $\gamma\delta$ T cells, NK cells, NK-like T cells, innate lymphoid cells, or MAIT cells) obtained by the any of the methods described above can be administered in a therapeutically effective amount to a subject (e.g., for treatment of cancer, e.g. for treatment of a solid tumor). In some cases, the therapeutically effective amount of engineered lymphocytes (e.g., $\gamma\delta$ T cells (e.g., engineered

$\gamma\delta$ T cells, blood-derived T cells, e.g., V$\delta$1 T cells, V$\delta$2 T cells, and/or DN T cells), NK cells, NK-like T cells, innate lymphoid cells, or MAIT cells) is less than $10 \times 10^{12}$ cells per dose (e.g., less than $9 \times 10^{12}$ cells per dose, less than $8 \times 10^{12}$ cells per dose, less than $7 \times 10^{12}$ cells per dose, less than $6 \times 10^{12}$ cells per dose, less than $5 \times 10^{12}$ cells per dose, less than $4 \times 10^{12}$ cells per dose, less than $3 \times 10^{12}$ cells per dose, less than $2 \times 10^{12}$ cells per dose, less than $1 \times 10^{12}$ cells per dose, less than $9 \times 10^{11}$ cells per dose, less than $8 \times 10^{11}$ cells per dose, less than $7 \times 10^{11}$ cells per dose, less than $6 \times 10^{11}$ cells per dose, less than $5 \times 10^{11}$ cells per dose, less than $4 \times 10^{11}$ cells per dose, less than $3 \times 10^{11}$ cells per dose, less than $2 \times 10^{11}$ cells per dose, less than $1 \times 10^{11}$ cells per dose, less than $9 \times 10^{10}$ cells per dose, less than $7.5 \times 10^{10}$ cells per dose, less than $5 \times 10^{10}$ cells per dose, less than $2.5 \times 10^{10}$ cells per dose, less than $1 \times 10^{10}$ cells per dose, less than $7.5 \times 10^{9}$ cells per dose, less than $5 \times 10^{9}$ cells per dose, less than $2.5 \times 10^{9}$ cells per dose, less than $1 \times 10^{9}$ cells per dose, less than $7.5 \times 10^{8}$ cells per dose, less than $5 \times 10^{8}$ cells per dose, less than $2,5 \times 10^{8}$ cells per dose, less than $1 \times 10^{8}$ cells per dose, less than $7.5 \times 10^{7}$ cells per dose, less than $5 \times 10^{7}$ cells per dose, less than $2,5 \times 10^{7}$ cells per dose, less than $1 \times 10^{7}$ cells per dose, less than $7.5 \times 10^{6}$ cells per dose, less than $5 \times 10^{6}$ cells per dose, less than $2,5 \times 10^{6}$ cells per dose, less than $1 \times 10^{6}$ cells per dose, less than $7.5 \times 10^{5}$ cells per dose, less than $5 \times 10^{5}$ cells per dose, less than $2,5 \times 10^{5}$ cells per dose, or less than $1 \times 10^{5}$ cells per dose).

In some embodiments, the therapeutically effective amount of engineered lymphocytes (e.g., $\gamma\delta$ T cells (e.g., engineered skin-derived $\gamma\delta$ T cells, engineered blood-derived $\gamma\delta$ T cells, e.g., V$\delta$1 T cells and/or DN T cells), NK cells, NK-like T cells, innate lymphoid cells, or MAIT cells) is less than $10 \times 10^{12}$ cells over the course of treatment (e.g., less than $9 \times 10^{12}$ cells, less than $8 \times 10^{12}$ cells, less than $7 \times 10^{12}$ cells, less than $6 \times 10^{12}$ cells, less than $5 \times 10^{12}$ cells, less than $4 \times 10^{12}$ cells, less than $3 \times 10^{12}$ cells, less than $2 \times 10^{12}$ cells, less than $1 \times 10^{12}$ cells, less than $9 \times 10^{11}$ cells, less than $8 \times 10^{11}$ cells, less than $7 \times 10^{11}$ cells, less than $6 \times 10^{11}$ cells, less than $5 \times 10^{11}$ cells, less than $4 \times 10^{11}$ cells, less than $3 \times 10^{11}$ cells, less than $2 \times 10^{11}$ cells, less than $1 \times 10^{11}$ cells, less than $9 \times 10^{10}$ cells, less than $7.5 \times 10^{10}$ cells, less than $5 \times 10^{10}$ cells, less than $2.5 \times 10^{10}$ cells, less than $1 \times 10^{10}$ cells, less than $7.5 \times 10^{9}$ cells, less than $5 \times 10^{9}$ cells, less than $2.5 \times 10^{9}$ cells, less than $1 \times 10^{9}$ cells, less than $7.5 \times 10^{8}$ cells, less than $5 \times 10^{8}$ cells, less than $2,5 \times 10^{8}$ cells, less than $1 \times 10^{8}$ cells, less than $7.5 \times 10^{7}$ cells, less than $5 \times 10^{7}$ cells, less than $2,5 \times 10^{7}$ cells, less than $1 \times 10^{7}$ cells, less than $7.5 \times 10^{6}$ cells, less than $5 \times 10^{6}$ cells, less than $2,5 \times 10^{6}$ cells, less than $1 \times 10^{6}$ cells, less than $7.5 \times 10^{5}$ cells, less than $5 \times 10^{5}$ cells, less than $2,5 \times 10^{5}$ cells, or less than $1 \times 10^{5}$ cells over the course of treatment).

In some embodiments, a dose of engineered lymphocytes (e.g., $\gamma\delta$ T cells, NK cells, NK-like T cells, innate lymphoid cells, or MAIT cells) as described herein includes about $1 \times 10^{6}$, $1.1 \times 10^{6}$, $2 \times 10^{6}$, $3.6 \times 10^{6}$, $5 \times 10^{6}$, $1 \times 10^{7}$, $1.8 \times 10^{7}$, $2 \times 10^{7}$, $5 \times 10^{7}$, $1 \times 10^{8}$, $2 \times 10^{8}$, or $5 \times 10^{8}$ cells/kg. In some embodiments, a dose of engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) includes at least about $1 \times 10^{6}$, $1.1 \times 10^{6}$, $2 \times 10^{6}$, $3.6 \times 10^{6}$, $5 \times 10^{6}$, $1 \times 10^{7}$, $1.8 \times 10^{7}$, $2 \times 10^{7}$ $5 \times 10^{7}$, $1 \times 10^{8}$, $2 \times 10^{8}$, or $5 \times 10^{8}$ cells/kg. In some embodiments, a dose of engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) includes up to about $1 \times 10^{6}$, $1.1 \times 10^{6}$, $2 \times 10^{6}$, $3.6 \times 10^{6}$, $5 \times 10^{6}$, $1 \times 10^{7}$, $1.8 \times 10^{7}$, $2 \times 10^{7}$, $5 \times 10^{7}$, $1 \times 10^{8}$, $2 \times 10^{8}$, or $5 \times 10^{8}$ cells/kg. In some embodiments, a dose of engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) includes about $1.1 \times 10^{6}$- $1.8 \times 10^{7}$ cells/kg. In some embodiments, a dose of engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) includes about $1 \times 10^{7}$, $2 \times 10^{7}$, $5 \times 10^{7}$, $1 \times 10^{8}$, $2 \times 10^{8}$, $5 \times 10^{8}$, $1 \times 10^{9}$, $2 \times 10^{9}$, or $5 \times 10^{9}$ cells. In some embodiments, a dose of engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) includes at least about $1 \times 10^{7}$, $2 \times 10^{7}$, $5 \times 10^{7}$, $1 \times 10^{8}$, $2 \times 10^{8}$, $5 \times 10^{8}$, $1 \times 10^{9}$, $2 \times 10^{9}$, or $5 \times 10^{9}$ cells. In some embodiments, a dose of engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) includes up to about $1 \times 10^{7}$, $2 \times 10^{7}$ $5 \times 10^{7}$, $1 \times 10^{8}$, $2 \times 10^{8}$, $5 \times 10^{8}$, $1 \times 10^{9}$, $2 \times 10^{9}$, or $5 \times 10^{9}$ cells.

In one embodiment, the subject is administered $10^{4}$ to $10^{6}$ engineered lymphocytes (e.g., $\gamma\delta$ T cells (e.g., skin-derived $\gamma\delta$ T cells, blood-derived $\gamma\delta$ T cells, e.g., V$\delta$1 T cells and/or DN T cells), NK cells, NK-like T cells, innate lymphoid cells, or MAIT cells) per kg body weight of the subject. In one embodiment, the subject receives an initial administration of a population of engineered lymphocytes (e.g., $\gamma\delta$ T cells, NK cells, NK-like T cells, innate lymphoid cells, or MAIT cells (e.g., an initial administration of $10^{4}$ to $10^{6}$ $\gamma\delta$ T cells, NK cells, NK-like T cells, innate lymphoid cells, or MAIT cells per kg body weight of the subject, e.g., $10^{4}$ to $10^{5}$ $\gamma\delta$ T cells, NK cells, NK-like T cells, innate lymphoid cells, or MAIT cells per kg body weight of the subject)), and one or more (e.g., 2, 3, 4, or 5) subsequent administrations of engineered lymphocytes (e.g., $\gamma\delta$ T cells, NK cells, NK-like T cells, innate lymphoid cells, or MAIT cells (e.g., one or more subsequent administration of $10^{4}$ to $10^{6}$ engineered $\gamma\delta$ T cells, NK cells, NK-like T cells, innate lymphoid cells, or MAIT cells per kg body weight of the subject, e.g., $10^{4}$ to $10^{5}$ engineered $\gamma\delta$ T cells per kg body weight of the subject)). In one embodiment, the one or more subsequent administrations are administered less than 15 days, e.g., 14, 13, 12, 11, 10, 9, 8, 7, 6, 5, 4, 3, or 2 days after the previous administration, e.g., less than 4, 3, or 2 days after the previous administration. In one embodiment, the subject receives a total of about $10^6$ $\gamma\delta$ T cells, NK cells, NK-like T cells, innate lymphoid cells, or MAIT cells per kg body weight of the subject over the course of at least three administrations of a population of $\gamma\delta$ T cells, NK cells, NK-like T cells, innate lymphoid cells, or MAIT cells, e.g., the subject receives an initial dose of 1 x $10^5$ $\gamma\delta$ T cells, NK cells, NK-like T cells, innate lymphoid cells, or MAIT cells, a second administration of 3 x $10^5$ $\gamma\delta$ T cells, NK cells, NK-like T cells, innate lymphoid cells, or MAIT cells, and a third administration of 6 x $10^5$ $\gamma\delta$ T cells, NK cells, NK-like T cells, innate lymphoid cells, or MAIT cells, and, e.g., each administration is administered less than 4, 3, or 2 days after the previous administration.

In some embodiments, one or more additional therapeutic agents can be administered to the subject. The additional therapeutic agent may be selected from the group consisting of an immunotherapeutic agent, a cytotoxic agent, a growth inhibitory agent, a radiation therapy agent, an anti-angiogenic agent, or a combination of two or more agents thereof. The additional therapeutic agent may be administered concurrently with, prior to, or after administration of the engineered lymphocytes (e.g., $\gamma\delta$ T cells, NK cells, NK-like T cells, innate lymphoid cells, or MAIT cells). The additional therapeutic agent may be an immunotherapeutic agent, which may act on a target within the subject's body (e.g., the subject's own immune system) and/or on the transferred $\gamma\delta$ T cells, NK cells, NK-like T cells, innate lymphoid cells, or MAIT cells.

The administration of the compositions may be carried out in any convenient manner. The compositions described herein may be administered to a patient transarterially, subcutaneously, intradermally, intratumorally, intranodally, intramedullary, intramuscularly, by intravenous injection, or intraperitoneally, e.g., by intradermal or subcutaneous injection. The compositions of engineered lymphocytes (e.g., $\gamma\delta$ T cells, NK cells, NK-like T cells, innate lymphoid cells, or MAIT cells) may be injected directly into a tumor, lymph node, or site of infection.

**EXAMPLES**

The following examples provide non-limiting methods for engineering $\gamma\delta$ T cells to express a heterologous targeting construct, functional screening of the engineered $\gamma\delta$ T cells, and therapeutic methods of using the engineered $\gamma\delta$ T cells.

**Example 1: Functional characterization of engineered $\gamma\delta$ T cell expressing a heterologous targeting construct**

Engineered V$\delta$1 T cells having a heterologous targeting receptor are functionally characterized in vitro by coculture with target cells (e.g., cancer cells, e.g., cells of a tumor cell line). Engineered V$\delta$1 T cells are compared against three control cell types: (1) untransduced V$\delta$1 T cells; (2) mock transduced V$\delta$1 T cells expressing heterologous GFP; and (3) conventional chimeric antigen receptor (CAR) transduced V$\delta$1 T cells having a functional intracellular signaling domain. Each group is cocultured with at least two groups of target tumor cells: (A) a healthy cell group expressing nominal levels of a tumor associated antigen (TAA), and (B) a tumor cell group expressing high levels of a TAA. Various effector-to-target ratios ($\gamma\delta$ T cell-to-target cell ratios) are tested. Untransduced or mock-transduced V$\delta$1 cells are used as a control to identify the effect conferred by the heterologous targeting receptor, and CAR T cells are used as a control to identify the effect of the lack of a functional intracellular domain configured to propagate a signal 1 and/or signal 2 stimulus. The following assays are performed:

1. A proliferation assay is performed according to a standard CFSE dilution protocol to quantify the effect of the interaction between engineered $\gamma\delta$ T cells and target cells on engineered $\gamma\delta$ T cell proliferation, which is indicative of activation against the target cell. $\gamma\delta$ T cells expressing a heterologous targeting construct proliferate to a greater degree in response to cancer cells relative to healthy cells.

2. A CD107 degranulation assay is performed by quantifying expression of lysosomal-associated membrane protein 1 (LAMP-1; i.e., CD107), which is expressed transiently on the surface of the $\gamma\delta$ T cells upon degranulation. Cells are stained at various time points to monitor the kinetics of degranulation. $\gamma\delta$ T cells expressing a heterologous targeting construct preferentially exhibit degranulation in response to cancer cells relative to healthy cells.

3. A perforin/granzyme assay is performed by staining for perforin and granzyme using FACS. $\gamma\delta$ T cells expressing a heterologous targeting construct preferentially express perforin and/or granzyme in response to cancer cells relative to healthy cells.

4. A cell lysis assay is performed to quantify the degree of target cell lysis (i.e., cytolysis). Kinetics of cell lysis are measured by Incucyte or luciferase assay as a percent of killing over time, and endpoint cell lysis is measured using a luciferase assay as the percent of killing at a given time point. $\gamma\delta$ T cells expressing a heterologous targeting construct preferentially lyse cancer cells relative to healthy cells.

5. Immunological synapse formation is monitored by live cell imaging. From observing the immunological synapse between $\gamma\delta$ T cells and target cells, binding kinetics are monitored. Additionally, calcium flux in $\gamma\delta$ T cells (indicating recognition) and PI blush in target cells (identifying a lethal hit) is observed. Target cell rounding is also observed. Binding kinetics and calcium flux is preferentially enhanced in $\gamma\delta$ T cells expressing a heterologous targeting construct when co-cultured with cancer cells, relative to healthy cells.

**Example 2: Peripheral blood-derived engineered $\gamma\delta$ T cell expressing a heterologous targeting construct**

One of the unique properties of V$\delta$1 $\gamma\delta$ T cells compared to conventional $\alpha\beta$ T cells is to selectively kill malignantly transformed cells whilst sparing healthy tissue, a process which can be mediated through the action of natural cytotoxicity receptors. The present results demonstrate that the ability of V$\delta$1 cells to eradicate tumour cells can be further enhanced using heterologous targeting constructs lacking intracellular signalling domains. Engineering V$\delta$1 cells with such constructs maintained or even increased the cytotoxicity of these cells towards malignantly transformed cells whilst still sparing healthy cells. This approach overcomes the observed on-target off-tumour effects of conventional chimeric antigen receptor (CAR) immunotherapy approaches, such as B-cell depletion following CD19 targeting CAR treatment.

Materials and Methods

*Peripheral blood $\gamma\delta$ T-cells isolation and expansion*

Blood derived V$\delta$1 cells were generated from peripheral blood of healthy donors, as previously described in U.S. 2018/0169147, which is hereby incorporated by reference in its entirety, in particular for its methods of isolating V$\delta$1 cells from blood. In brief, MACS-depleted $\alpha\beta$ T cells were resuspended in serum-free culture medium (CTS OpTmizer) supplemented with autologous plasma and expanded in presence of IL-4, IFN-$\gamma$, IL-21, IL-1$\beta$, IL-15, and soluble OKT3. Cells were transduced with lentiviral vector encoding the constructs described below. Essentially, the full-length CAR constructs included an scFv binder region targeting the tumour antigen CD19 or GD2, a transmembrane domain, and an intracellular signalling domain (according to conventional CAR construct design). The nonsignaling or "nsCAR" construct lacked the intracellular domain.

Other means for obtaining Vd1 cells from blood are well known in the art, such as US 9499788, WO2017197347, WO2016081518. Alternatively, V$\delta$1 cells are isolated from human skin biopsies as described in U.S. 2018/0312808, which is hereby incorporated by reference in its entirety and specifically for methods of isolating V$\delta$1 cells from tissue. Skin-derived V$\delta$1 cells are transduced as above.

*Flow Cytometry analysis*

Immunophenotyping was conducted using a BD FACS Lyric flow cytometer. Cells were analysed for the expression of surface markers using a PerCP-Vio700 anti-TCR a/b (Miltenyi), APC anti-TCR g/d (Miltenyi), VioBlue anti-TCR V$\delta$1 (Miltenyi), PE anti-NKp30 (BioLegend), APC anti NKp44 (BioLegend), PerCP.Cy5.5 anti-NKG2D (BioLegend). Conventional CD19 CAR and nonsignaling CD19 CAR construct expression was detected with a FITC anti-STREP tag antibody (LSBio). NonsignallingGD2 CAR expression was monitored using PE anti-FC antibody (BioLegend).

*Cytotoxicity assay*

Nalm-6 (ATCC, CRL-1567) and primary B cells were labelled with CTV or CFSE and combined with T cells at 1:1 effector-to-target ratio. Cultures were incubated for 16 hours at 37°C. Following incubation, SytoxAADvanced (Invitrogen) and absolute counting beads were added to the wells and flow cytometry acquisition was performed. Cytotoxicity was calculated as follows:

$$100 - (sample\ counts/maximum\ counts) \times 100$$

where the maximum count is the number of target cells in the absence of any effector cells.

*Live-cell imaging*

Human GD2 expressing neuroblastoma cell line Kelly (DSMZ ACC-355) was stably transduced with NucLight Green encoding lentiviral vector (Essen BioScience) to enable automated cell counting. Cell growth was monitored using Incucyte Zoom Live-Cell Imaging System (Essen Bioscience) for 60 hours in one-hour intervals. Data were expressed as the change in ratio of the number of green object-count per image at given time point normalised to the number of green object-count per image at time zero. Each data point represents triplicate wells.

*Comparison with $\alpha\beta$ T cell CAR*

The $\gamma\delta$ cells were engineered with full length or nonsignaling CAR constructs as above. Similarly, $\alpha\beta$-derived T cells from blood or tissue are also engineered with full-length or nonsignalling CAR constructs. The engineered cells are measured for cytolytic activity against healthy and malignant cells that express the target antigen to demonstrate the on-target off-tumour cytotoxicity of each population.

Results

Transduction of blood-derived V$\delta$1 cells with lentiviral vector encoding for full length (CAR19) or nonsignalling anti-CD19 targeting constructs resulted in greater than 90% transduction efficiency (FIG. 3A). There was no significant difference in the surface expression of CAR molecules. Neither the immunophenotype, nor the proliferative capacity of the transduced cells was altered by lentiviral transduction. FACS analysis of untransduced (UTD) and transduced V$\delta$1 cells did not reveal any significant difference in the surface expression of key natural cytotoxicity receptor (NCR) molecules (NKp30, NKp44, and NKG2D; FIG. 3B).

V$\delta$1 cells recognized and killed cells of the CD19 expressing acute lymphoid leukaemia cell line NALM-6. Expression of either full length or nonsignalling CD19 CAR on V$\delta$1 cells resulted in a two-fold increase in target cell killing (FIGS. 4A and 4B; percentage of killing 21.4% (UTD) vs. 46% (nsCAR19) vs. 58% (CAR19) and 42.8% (UTD) vs. 83% (nsCAR19) vs. 88% (CAR19) for donor 1 and donor 2, respectively, at a 1:1 effector to target ratio). Importantly, nonsignalling anti-CD19 CAR expressing V$\delta$1 cells did not kill healthy human B cells (FIG. 4C).

To further prove the general applicability of the nonsignalling CAR approach, V$\delta$1 cells were redirected towards tumour cells expressing GD2 antigen. Transduction of blood-derived V$\delta$1 cells with GD2 specific nsCAR molecule resulted in a 54% transduction efficiency measured by FACS (FIG. 5A). Untransduced and nsCAR transduced V$\delta$1 cells were co-cultured with GD2 expressing neuroblastoma cell line (Kelly) at a 1:1 effector to target ratio. Target cell killing was measured using live-cell imaging (Incucyte, Essen Bioscience). Co-culture of Kelly cells with nsCAR expressing V$\delta$1 cells resulted in a 40% reduction in total target cell numbers compared to targets cells cultured in the presence of untransduced V$\delta$1 cells (FIG. 5B).

## Example 3: Treating cancer with a $\gamma\delta$ T cell engineered with a heterologous targeting construct

A heterologous targeting construct is synthesized using cloning and PCR methods known in the art. A protein fragment encoding an scFv that targets a tumor-associated antigen (TAA) is fused to the N-terminus of a stalk domain, which is fused to the N-terminus of a CD8 transmembrane domain. The heterologous targeting construct is then cloned into a lentiviral vector.

A patient undergoes a leukapheresis procedure where a blood sample is obtained and red blood cells are depleted. $\alpha\beta$ T cells are depleted using standard magnetic separation protocols. The remaining population, which includes $\gamma\delta$ T cells, is expanded using any suitable method of $\gamma\delta$ T cell expansion known in the art or described herein. During expansion, cells are incubated with the lentiviral vector containing the polynucleotide encoding the heterologous targeting construct, and the polynucleotide is integrated into the genome of the $\gamma\delta$ T cells by reverse transcription. The cells transduced with the lentiviral vector will express the heterologous targeting construct on the surface. The transduced cells expressing the heterologous targeting construct are then separated from the non-transduced cells and harvested for infusion as an autologous or allogeneic therapy.

The cells are administered intravenously to the patient over a course of two hours. The intravenous administration is repeated once per week for 12 weeks and symptoms of the cancer are monitored.

## Other Embodiments

All publications, patents, and patent applications mentioned in this specification are herein incorporated by reference to the same extent as if each independent publication or patent application was specifically and individually indicated to be incorporated by reference.

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth, and follows in the scope of the claims.

Other embodiments are within the claims. What is claimed is:

The following numbered clauses, describing aspects of the invention, are part of the description.

1. An engineered gamma-delta (γδ) T cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered γδ T cell.

2. The engineered γδ T cell of clause 1, further comprising a stalk domain operatively linking the antigen-binding domain to the transmembrane domain.

3. An engineered γδ T cell comprising a heterologous targeting construct, wherein the heterologous targeting construct comprises an antigen-binding domain and a transmembrane domain, wherein the transmembrane domain is a terminal transmembrane domain that does not propagate signal 1 activation of the engineered γδ T cell.

4. The engineered γδ T cell of clause 3, further comprising a stalk domain operatively linking the antigen-binding domain to the transmembrane domain.

5. An engineered γδ T cell comprising a heterologous targeting construct, wherein the heterologous targeting construct consists of an antigen-binding domain, a stalk domain operatively linked the antigen-binding domain, and a transmembrane domain operatively linked to the stalk domain, wherein the heterologous targeting construct does not propagate signal 1 activation of the engineered γδ T cell.

6. The engineered γδ T cell of any one of clauses 3-5, wherein the transmembrane domain does not activate the engineered γδ T cell.

7. The engineered γδ T cell of any one of clauses 1-6, wherein the engineered γδ T cell is Vδ2-negative.

8. The engineered γδ T cell of clause 6, wherein the Vδ2-negative γδ T cell is Yδ1-positive.

9. The engineered γδ T cell of any one of clauses 1-8, wherein the antigen-binding domain comprises a single chain variable fragment (scFv), a monoclonal antibody, a Fab fragment, a B cell receptor, a T cell receptor, an antibody scaffold, a receptor-specific ligand, or a ligand-specific receptor.

10. The engineered γδ T cell of any one of clauses 2 or 4-9, wherein the stalk domain comprises one or more of the domains selected from the group consisting of a CD8 stalk, an IgG1 hinge, an IgG1 hinge-$CH_2$ domain, an IgG1-hinge-$CH_3$ domain, an IgG1-hinge-$CH_2$- $CH_3$ domain, a $(G_4S)_3$ hinge, an a CD7 stalk, an IgD hinge, an IgD hinge-$CH_2$ domain, an IgD hinge-$CH_2$-$CH_3$ domain, an IgD hinge-$CH_3$ domain, an IgG4 hinge, an IgG4 hinge-$CH_2$ domain, an IgG4 hinge-$CH_2$-$CH_3$ domain, an IgG4 hinge-$CH_3$ domain, or an FcεRI stalk.

11. The engineered γδ T cell of any one of clauses 1-10, wherein the transmembrane domain comprises a CD8 transmembrane domain, a CD4 transmembrane domain, a CD3ζ transmembrane domain, a CD28 transmembrane domain, a CD45 transmembrane domain, a CD5 transmembrane domain, a CD8 transmembrane domain, a CD9 transmembrane domain, a CD16 transmembrane domain, a CD22 transmembrane domain, a CD33 transmembrane domain, a CD37 transmembrane domain, a CD64 transmembrane domain, a CD80 transmembrane domain, a CD86 transmembrane domain, a CD134 transmembrane domain, a CD137 transmembrane domain, a CD154 transmembrane domain, a CD7 transmembrane domain, a CD71 transmembrane domain, a CD18 transmembrane domain, a CD29 transmembrane domain, a CD11a transmembrane domain, a CD11b transmembrane domain, a CD11c transmembrane domain, a CD11d transmembrane domain, a CD94 transmembrane domain, an FcγR transmembrane domain, or an NKG2D transmembrane domain.

12. The engineered γδ T cell of any one of clauses 1-11, wherein no more than 50% of the amino acids of the C-terminal transmembrane domain reside intracellularly.

13. The engineered γδ T cell of any one of clauses 1-12, wherein clustering of the heterologous targeting construct upon binding of the antigen-binding domain to a target antigen does not substantially activate the TCR pathway in the engineered γδ T cell.

14. The engineered γδ T cell of any one of clauses 1-13, wherein the antigen-binding domain binds a tumor-associated antigen.

15. The engineered γδ T cell of clause 14, wherein the tumor-associated antigen is a protein or peptide antigen expressed on the surface of a tumor cell.

16. The engineered γδ T cell of clause 15, wherein the tumor-associated antigen is CD19.

17. The engineered γδ T cell of clause 16, wherein the tumor-associated antigen is a carbohydrate expressed on the surface of a tumor cell.

18. The engineered γδ T cell of clause 14, wherein the tumor-associated antigen is ganglioside expressed on the surface of a tumor cell.

19. The engineered γδ T cell of clause 18, wherein the ganglioside is GD2.

20. The engineered γδ T cell of any one of clauses 14-19, wherein the tumor-associated antigen is an immunosuppressive antigen.

21. The engineered γδ T cell of any one of clauses 1-20, wherein the antigen-binding domain binds a target antigen that is expressed by a solid tumor cell.

22. The engineered γδ T cell of any one of clauses 1-21, wherein binding of the antigen-binding domain to a target antigen expressed on a healthy cell triggers substantially less cytolysis by the engineered γδ T cell relative to a reference cell having a functional intracellular domain.

23. The engineered γδ T cell of clause 22, wherein binding of the antigen-binding domain to the target antigen expressed on a healthy cell does not substantially trigger cytolysis by the engineered γδ T cell.

24. The engineered γδ T cell of any one of clauses 1-23, wherein binding of the antigen-binding domain to a target antigen expressed on a tumor cell or an infected cell substantially triggers cytolysis by the engineered γδ T cell.

25. The engineered γδ T cell of clause 22, wherein the cytolysis is dependent on endogenous expression of NKG2D, NKp30, NKp44, NKp46, or DNAM1 by the engineered γδ T cell.

26. The engineered γδ T cell of clause 24 or 25, wherein the cytolysis is characterized by one, two, three, four, five, or all six of the responses selected from the group consisting of CD107 degranulation, granzyme release, perforin release, granulysin release, target cell killing, proliferation of the γδ T cell, and cytokine production.

27. An engineered NK cell or NK-like T cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered NK cell or NK-like T cell.

28. An engineered innate lymphoid cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered innate lymphoid cell.

29. An engineered mucosal-associated invariant T (MAIT) cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered mucosal-associated invariant T cell.

30. An isolated cell population, the population comprising at least ten engineered γδ T cells of any one of clauses 1-26, engineered NK cells or NK-like T cells of clause 27, engineered innate lymphoid cells of clause 28, or engineered MAIT cells of clause 29.

31. The isolated cell population of clause 30, wherein the engineered $\gamma\delta$ T cells, the engineered NK cells or NK-like T cells, the engineered innate lymphoid cells, or engineered MAIT cells represent greater than 2% of the total number of cells in the isolated cell population.

32. An isolated cell population, the population comprising a population of the engineered $\gamma\delta$ T cells of any one of clauses 1-26, a population of the engineered NK cells or NK-like T cells of clause 27, a population of the engineered innate lymphoid cells of clause 28, or a population of the engineered MAIT cells of clause 29, wherein the population represents greater than 2% of the total number of cells in the isolated cell population.

33. The isolated cell population of clause 31 or 32, comprising at least ten engineered $\gamma\delta$ T cells of any one of clauses 1-26, and/or at least ten engineered NK cells or NK-like T cells of clause 27, and/or at least ten engineered innate lymphoid cells of clause 28, and/or at least ten engineered MAIT cells of clause 29.

34. A $\gamma\delta$ T cell comprising a heterologous polynucleotide, the polynucleotide encoding heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered $\gamma\delta$ T cell.

35. A $\gamma\delta$ T cell comprising a heterologous polynucleotide, the polynucleotide encoding a targeting construct, wherein the heterologous targeting construct comprises an antigen-binding domain and a transmembrane domain, wherein the transmembrane domain is a terminal transmembrane domain that does not participate in signal 1 activation of the engineered $\gamma\delta$ T cell.

36. The engineered $\gamma\delta$ T cell of any one of clauses 1-26, the engineered NK cell or NK-like T cell of clause 27, the engineered innate lymphoid cell of clause 28, the engineered MAIT cell of clause 29, the isolated cell population of any one of clauses 30-33, or the $\gamma\delta$ T cell comprising a heterologous polynucleotide of clause 34 or 35, for use in a method of treating a subject by adoptive T cell therapy, wherein the method comprises administering a therapeutically effective amount of the engineered $\gamma\delta$ T cells of any one of clauses 1-24, the engineered NK cell or NK-like T cell of clause 25, the engineered innate lymphoid cell of clause 26, the engineered MAIT cell of clause 27, the isolated cell population of any one of clauses 28-31, or the $\gamma\delta$ T cells comprising a heterologous polynucleotide of clause 32 or 33, to a subject in need thereof.

37. The engineered $\gamma\delta$ T cell, engineered NK cell or NK-like T cell, engineered innate lymphoid cell, engineered MAIT cell, isolated cell population, or $\gamma\delta$ T cell comprising a heterologous polynucleotide for use according to clause 36, wherein the subject is a human.

38. The engineered $\gamma\delta$ T cell, engineered NK cell or NK-like T cell, engineered innate lymphoid cell, engineered MAIT cell, isolated cell population, or $\gamma\delta$ T cell comprising a heterologous polynucleotide for use according to clause 37, wherein the human is a human cancer patient.

39. The engineered $\gamma\delta$ T cell, engineered NK cell or NK-like T cell, engineered innate lymphoid cell, engineered MAIT cell, isolated cell population, or $\gamma\delta$ T cell comprising a heterologous polynucleotide for use according to clause 38, wherein the human cancer patient is being treated for a solid tumor.

40. The engineered $\gamma\delta$ T cell, engineered NK cell or NK-like T cell, engineered innate lymphoid cell, engineered MAIT cell, isolated cell population, or $\gamma\delta$ T cell comprising a heterologous polynucleotide for use according to clause 37, wherein the human is a human patient being treated for a viral infection.

41. A method of treating a subject by adoptive T cell therapy, wherein the method comprises administering a therapeutically effective amount of the engineered $\gamma\delta$ T cells of any one of clauses 1-26, the engineered NK cell or NK-like T cell of clause 27, the engineered innate lymphoid cell of clause 28, the engineered MAIT cell of clause 29, the isolated cell population of any one of clauses 30-33, or the $\gamma\delta$ T cells comprising a heterologous polynucleotide of clause 34 or 35, to a subject in need thereof.

42. The method of clause 41, wherein the subject is a human.

43. The method of clause 42, wherein the human is a human cancer patient.

44. The method of clause 43, wherein the human cancer patient is being treated for a solid tumor.

45. The method of clause 42, wherein the human is a human patient being treated for a viral infection.

**Claims**

1. An engineered NK cell or NK-like T cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered NK cell or NK-like T cell.

2. An engineered innate lymphoid cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered innate lymphoid cell.

3. An engineered mucosal-associated invariant T (MAIT) cell comprising a heterologous targeting construct, wherein the heterologous targeting construct comprises an extracellular antigen-binding domain and a transmembrane domain operatively linked to the antigen-binding domain, wherein the heterologous targeting construct lacks an intracellular domain capable of activating the engineered mucosal-associated invariant T cell.

4. The engineered NK cell or NK-like T cell of claim 1, the engineered innate lymphoid cell of claim 2, or the engineered MAIT cell of claim 3, wherein (i) the transmembrane domain does not participate in an intracellular signaling pathway, (ii) binding of the antigen-binding domain to a target antigen expressed on a healthy cell triggers substantially less cytolysis by the engineered $\gamma\delta$ T cell relative to a reference cell having a functional intracellular domain and/or (iii) binding of the antigen-binding domain to a target antigen expressed on a tumor cell or an infected cell substantially triggers cytolysis by the engineered $\gamma\delta$ T cell.

5. The engineered NK cell or NK-like T cell, the engineered innate lymphoid cell or the engineered MAIT cell of claim 4, wherein the cytolysis is dependent on endogenous expression of NKG2D, NKp30, NKp44, NKp46, or DNAM1 by the engineered cell.

6. The engineered NK cell or NK-like T cell, the engineered innate lymphoid cell or the engineered MAIT cell of claim 4 or 5, wherein the cytolysis is **characterized by** one, two, three, four, five, or all six of the responses selected from the group consisting of CD107 degranulation, granzyme release, perforin release, granulysin release, target cell killing, proliferation of the $\gamma\delta$ T cell, and cytokine production.

7. The engineered NK cell or NK-like T cell of any one of claims 1 and 4-6, the engineered innate lymphoid cell of any one of claims 2 and 4-6, or the engineered MAIT cell of any one of claims 3-6, wherein the antigen-binding domain comprises a single chain variable fragment (scFv), a monoclonal antibody, a Fab fragment, a B cell receptor, a T cell receptor, an antibody scaffold, a receptor-specific ligand, or a ligand-specific receptor.

8. The engineered NK cell or NK-like T cell of any one of claims 1 and 4-7, the engineered innate lymphoid cell of any one of claims 2 and 4-7, or the engineered MAIT cell of any one of claims 3-7, further comprising a stalk domain operatively linking the antigen-binding domain to the transmembrane domain.

9. The engineered NK cell or NK-like T cell of any one of claims 1 and 4-8, the engineered innate lymphoid cell of any one of claims 2 and 4-8, or the engineered MAIT cell of any one of claims 3-8, wherein the stalk domain comprises one or more of the domains selected from the group consisting of a CD8 stalk, an IgG1 hinge, an IgG1 hinge-CH2 domain, an IgG1-hinge-CH3 domain, an IgG1-hinge-CH2-CH3 domain, a (G4S)3 hinge, an a CD7 stalk, an IgD hinge, an IgD hinge-CH2 domain, an IgD hinge-CH2-CH3 domain, an IgD hinge-CH3 domain, an IgG4 hinge, an IgG4 hinge-CH2 domain, an IgG4 hinge-CH2-CH3 domain, an IgG4 hinge-CH3 domain, or an FcεRI stalk.

10. The engineered NK cell or NK-like T cell of any one of claims 1 and 4-9, the engineered innate lymphoid cell of any one of claims 2 and 4-9, or the engineered MAIT cell of any one of claims 3-9, wherein the transmembrane domain comprises a CD8 transmembrane domain, a CD4 transmembrane domain, a CD3ζ transmembrane domain, a CD28 transmembrane domain, a CD45 transmembrane domain, a CD5 transmembrane domain, a CD8 transmembrane domain, a CD9 transmembrane domain, a CD16 transmembrane domain, a CD22 transmembrane domain, a CD33 transmembrane domain, a CD37 transmembrane domain, a CD64 transmembrane domain, a CD80 transmembrane domain, a CD86 transmembrane domain, a CD134 transmembrane domain, a CD137 transmembrane domain, a CD154 transmembrane domain, a CD7 transmembrane domain, a CD71 transmembrane domain, a CD18 transmembrane domain, a CD29 transmembrane domain, a CD11a transmembrane domain, a CD11b transmembrane domain, a CD11c transmembrane domain, a CD11d transmembrane domain, a CD94 transmembrane domain, an FcγR transmembrane domain, or an NKG2D transmembrane domain.

11. The engineered NK cell or NK-like T cell of any one of claims 1 and 4-10, the engineered innate lymphoid cell of any one of claims 2 and 4-10, or the engineered MAIT cell of any one of claims 3-10, wherein the antigen-binding domain binds a tumor-associated antigen, such as CD19 or GD2.

12. An isolated cell population comprising at least ten engineered NK cells or NK-like T cells of any one of claims 1 and 4-11, and/or at least ten engineered innate lymphoid cells of any one of claims 2 and 4-11, and/or at least ten engineered MAIT cells of any one of claims 3-11.

13. The isolated cell population of claim 12, wherein the engineered NK cells or NK-like T cells, the engineered innate lymphoid cells, or engineered MAIT cells represent greater than 2% of the total number of cells in the isolated cell population.

14. The engineered NK cell or NK-like T cell of any one of claims 1 and 4-11, the engineered innate lymphoid cell of any one of claims 2 and 4-11, the engineered MAIT cell of any one of claims 3-11, or the isolated cell population of claim 12 or 13, for use in a method of treating a subject by adoptive T cell therapy, wherein the method comprises administering a therapeutically effective amount of the engineered NK cell or NK-like T cell of any one of claims 1 and 4-11, the engineered innate lymphoid cell of any one of claims 2 and 4-11, the engineered MAIT cell of any one of claims 3-11, or the isolated cell population of claim 12 or 13, to a subject in need thereof.

15. The engineered NK cell or NK-like T cell, engineered innate lymphoid cell, engineered MAIT cell, or isolated cell population for use according to claim 14, wherein the subject is a human.

16. The engineered NK cell or NK-like T cell, engineered innate lymphoid cell, engineered MAIT cell, or isolated cell population for use according to claim 15, wherein the human is a human cancer patient, e.g. a human cancer patient is being treated for a solid tumor, or wherein the human is a human patient being treated for a viral infection.

# FIGURE 1

# FIGURE 2

A.                    B.                    C.

# FIGURE 3

**A**

**B**

# FIGURE 4

# FIGURE 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5786464 A
- US 6114148 A
- EP 239400 A
- WO 9109967 A
- US 5225539 A
- US 5530101 A
- US 5585089 A
- EP 592106 A
- EP 519596 A
- US 5565332 A
- US 20050042664
- US 20050048617
- US 6407213 B
- US 5766886 A
- WO 9317105 A

- US 20050100543 A
- US 20050175606 A
- US 20070014794 A
- WO 2006020258 A
- WO 2007024715 A
- US 20180312808 A
- US 9499788 B
- WO 2016198480 A
- WO 2017197347 A
- WO 2016081518 A
- US 20160175338 A
- GB 1707048 A
- WO 2018202808 A
- WO 2017072367 A
- US 20180169147 A

**Non-patent literature cited in the description**

- **DENIGER et al.** *Frontiers in Immunology,* 2014, vol. 5 (636), 1-10
- **VAZQUZ-LOMBARDI et al.** *Drug Discovery Today,* 2015, vol. 20 (10), 1271-83
- Harrison's Principles of Internal Medicine. Mc-Graw-Hill, 2012
- **GHOSH et al.** *Nat. Med.,* 2017, vol. 23, 242-251
- **WHILDING et al.** *Mol. Ther.,* 2017, vol. 25, 259-273
- **WILKIE et al.** *J. Biol. Chem.,* 2010, vol. 285, 25538-25544

- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **BIRD et al.** *Science,* 1988, vol. 242, 423-426
- **HUSTON et al.** *Proc. Natl. Acad. Sci. USA,* 1988, vol. 85, 5879-5883
- **HOLLINGER et al.** *Proc Natl Acad. Sci. USA,* 1993, vol. 90, 6444-6448
- **ALMEIDA et al.** *Clinical Cancer Research,* 2016, vol. 22 (23), 5795-5805